# EUROPÄISCHE PATENTSCHRIFT

(11) **EP 0 455 013 B1**
(45) Veröffentlichungstag und Bekanntmachung des Hinweises auf die Patenterteilung: **21.06.1995**
(21) Anmeldenummer: 91105715.6
(22) Anmeldetag: 11.04.1991
(51) Int. Cl.: C07D 475/04

(54) **Ammoniumsalze von N(5)methyl-5,6,7,8-tetrahydrofolsäure**
Ammoniumsalts of N(5)-methyl-5,6,7,8-tetrahydrofolic acid
Sels d'ammonium de l'acide N(5)-méthyl-5,6,7,8-tétrahydrofolique

(30) Priorität: 12.04.1990 CH 1255/90
(43) Veröffentlichungstag der Anmeldung: 06.11.1991
(73) Patentinhaber: Cerbios-Pharma S.A., CH-6917 Barbengo (CH)
(72) Erfinder: Marazza, Fabrizio, CH-6921 Vico Morcote (CH); Jacques, Jean, F-75005 Paris (FR)
(74) Vertreter: Zink, Markus, Dr.

(56) Entgegenhaltungen:
- EP-A- 0 266 042
- EP-A- 0 293 029
- EP-A- 0 348 641
- DE-A- 2 063 027
- US-A- 2 688 018

## Beschreibung

Die vorliegende Erfindung betrifft Ammoniumsalze von N⁵-Methyl-5,6,7,8-tetrahydrofolsäure und Verfahren zu ihrer Herstellung. Diese Erfindung betrifft auch ein Verfahren zur Trennung des (6RS)-Diastereoisomerengemisches der Ammoniumsalze von N⁵-Methyl-5,6,7,8-tetrahydrofolsäure in die einzelnen (6R) und (6S)-Diasteroisomeren, und deren Umwandlung zu den entsprechenden (6R) oder (6S)-Alkalimetall- oder Erdalkalimetallsalzen.

N⁵-Methyl-5,6,7,8-tetrahydrofolsäure, hierin teilweise mit der Bezeichnung N⁵-Methyl-THF abgekürzt, ist die vorherrschende zirkulierende Form von reduzierten Folaten in Säugern.

Es besteht ein zunehmendes Interesse für die Anwendung dieses natürlichen Metaboliten als wenigstens eine aktive Verbindung in einem therapeutischen Mittel, beispielsweise als Vitamin bei Folatmangelzuständen. Therapeutische Mittel, enthaltend N⁵-Methyl-THF, können auch verwendet werden für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen. N⁵-Methyl-THF ist in einem solchen Mittel gewöhnlich in einer Menge von 1 mg bis 500 mg, insbesondere von 5 mg bis 150 mg, pro Dosiseinheit enthalten. Das Arzneimittel liegt vorzugsweise in Form eines parenteralen und/oder oralen pharmazeutischen Präparates vor. Es wird auf die am Schluss der Beschreibung erwähnten Referenzen Nr. 1 bis 5 verwiesen.

Die absolute Konfiguration der natürlichen Form von N⁵-Methyl-THF entspricht der folgenden Formel A 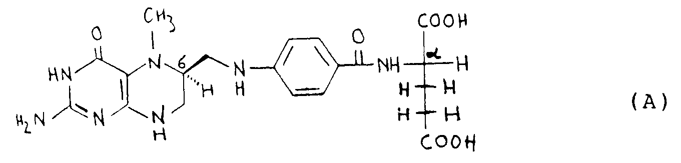
und wird als (6S,L) definiert, wobei sich "6S" auf die Konfiguration am C₆ des Pterin-Ringsystems und "L" auf die Konfiguration des α-Kohlenstoffatoms der Glutamat-Seitenkette beziehen.

Im Handel erhältliche Formen von N⁵-Methyl-THF, erhalten aus der chemischen Reduktion von Folsäure, sind ein (6RS)-Diastereoisomerengemisch mit der "L"-Konfiguration in der Glutamatseitenkette.
Ueber die Funktion des (6R)-Diastereoisomeren von N⁵-Methyl-THF ist man sich nicht einig. Es ist angenommen worden, dass das unnatürliche (6R)-Diastereoisomer von N⁵-Methyl-THF inert ist und als solches unverändert ausgeschieden wird; siehe beispielsweise die Referenz Nr. 13. Es ist jedoch auch postuliert worden, dass das unnatürliche (6R)-Diastereoisomer auf das Folattransportsystem durch die Zellmembran in Säugern, einschliesslich Menschen, interferieren könnte; siehe beispielsweise die Referenzen Nr. 6 bis 8.

Die individuellen (6S) und (6R) Diastereoisomeren von N⁵-Methyl-THF sind in Milligramm-Mengen von fragwürdiger chemischer Reinheit hergestellt worden, entweder durch komplexe enzymatische Reaktionen (siehe beispielsweise Referenz Nr. 6), oder indirekt durch Reduktion des entsprechenden N⁵, N¹⁰-Methylen-THF Isomers, welches erhalten wurde durch chromatographische Trennung des (6RS)-Gemisches (siehe die Referenzen Nr. 7 und 9).

5,6,7,8-Tetrahydrofolsäure wird hierin teilweise mit der Bezeichnung THF abgekürzt.

Kürzlich sind indirekte Verfahren zur Herstellung der individuellen Diastereoisomeren von N⁵-Methyl-THF publiziert worden; siehe die Referenzen Nr. 10 und 11. Diese Verfahren basieren auf der fraktionierten Kristallisation von N⁵, N¹⁰-Methenyl-THF.Cl^{⊖}.HCl der Formel 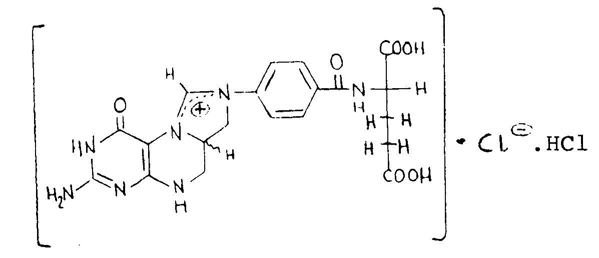
beziehungsweise von N⁵, N¹⁰-Methenyl-THF.Cl^{⊖} der Formel 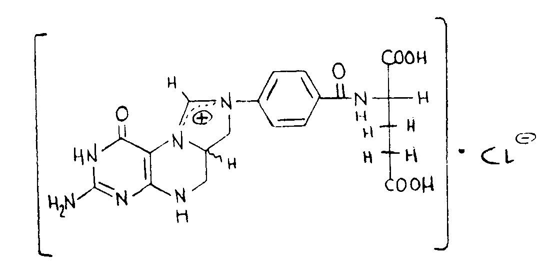
gefolgt von der chemischen Umwandlung der getrennten, einzelnen (6R) und (6S) Diastereoisomeren in N⁵-Methyl-THF. Obwohl diese Verfahren in industriellem Massstab durchführbar sind, haben sie den Nachteil, dass sie mehrere chemische Schritte umfassen, was sie ökonomisch wenig attraktiv macht.

Es ist eine Aufgabe der vorliegenden Erfindung, ein einfaches, billiges und effizientes Verfahren zur Verfügung zu stellen, mit welchem man ein (6RS)-Diastereoisomerengemisch eines N⁵-Methyl-THF-Derivates in die reinen, einzelnen (6R) und (6S)-Diastereoisomeren auftrennen kann.

Völlig überraschend wurde gefunden, dass diese Aufgabe erfindungsgemäss mittels fraktionierter Kristallisation von neuen Ammoniumsalzen eines (6RS)-Diastereoisomerengemisches von N⁵-Methyl-THF gelöst werden kann.

Nebst diesem erfindungsgemässen Trennungsverfahren und diesen erfindungsgemässen Ammoniumsalzen werden auch zwei erfindungsgemässe Verfahren zur Herstellung der genannten Ammoniumsalze zur Verfügung gestellt. Ferner wird auch noch ein erfindungsgemässes Verfahren zur Herstellung von Alkalimetall- oder Erdalkalimetallsalzen von (6R) oder (6S) N⁵-Methyl-THF aus einem entsprechenden erfindungsgemässen diastereoisomerenreinen Ammoniumsalz beschrieben.

Die vorliegende Erfindung ist durch die Merkmale in den unabhängigen Ansprüchen gekennzeichnet. Bevorzugte Ausführungsformen dieser Erfindung sind in den abhängigen Ansprüchen definiert.

Im Folgenden werden verschiedene mögliche Ausführungsformen beschrieben. Dabei werden Ausführungsformen, wie sie in den Ansprüchen definiert sind, normalerweise nicht mehr wiederholt.

Beim ersten erfindungsgemässen Verfahren zur Herstellung der erfindungsgemässen Ammoniumsalze wird N⁵-Methyl-THF vorzugsweise in der Form des Calciumsalzes in Wasser vorgelegt. Die Stickstoff enthaltende Base, welche dann hinzugegeben wird, kann ein primäres oder sekundäres Alkyl- oder Arylamin sein. Das vorhandene Calciumkation wird zweckmässig durch die Hinzugabe von Oxalsäure als Calciumoxalat gefällt, welches anschliessend abfiltriert wird. Das wässrige Filtrat enthält das gelöste Ammoniumsalz. Nach der Verdampfung des Wassers unter vermindertem Druck oder nach der Lyophilisation wird das Ammoniumsalz erhalten.

Beim zweiten erfindungsgemässen Verfahren zur Herstellung der erfindungsgemässen Ammoniumsalze wird N⁵-Methyl-THF, also in Form der Carbonsäure, zweckmässigerweise in Wasser oder in einem Gemisch aus Wasser und Ethanol und/oder Methanol vorgelegt. Bei diesem Verfahren entfallen die Schritte der Fällung des Alkalimetall- oder Erdalkalimetallkations und deren Abtrennung, wie dies beim ersten erfindungsgemässen Verfahren beschrieben worden ist. Die übrigen Schritte des zweiten erfindungsgemässen Verfahrens entsprechen den entsprechenden Schritten im ersten erfindungsgemässen Verfahren.

Die erfindungsgemässen Ammoniumsalze können auch noch mittels Ionenaustausch auf einer entsprechenden Säule hergestellt werden, auf welcher beispielsweise ein Calciumkation gegen das gewünschte Ammoniumion ausgetauscht wird.

Die erfindungsgemässen Ammoniumsalze, beispielsweise gemäss einem oben beschriebenen Verfahren hergestellt, werden nun einer fraktionierten Kristallisation unterworfen, bei der das (6RS)-Diastereoisomerengemisch in die einzelnen, reinen (6R) und (6S) Diastereoisomeren aufgetrennt wird. Dazu wird vorzugsweise ein Gewichtsteil eines erfindungsgemässen Ammoniumsalzes mit 5 Gewichtsteilen, bezogen auf obigen Gewichtsteil an Ammoniumsalz, eines Gemisches aus 85 Vol.-% Ethanol und 15 Vol.-% Wasser vermischt. In Abhängigkeit des Ammoniumsalzes kann eine Erwärmung auf eine Temperatur von 40°C bis 60°C notwendig sein, um eine klare Lösung zu erhalten.
Aus dieser klaren Lösung lässt man ein Diastereoisomer auskristallisieren und filtriert es anschliessend ab. Die Kristallisation kann bei Eiswassertemperatur oder im Kühlschrank oder bei einer Temperatur von 10°C bis 25°C erfolgen. Falls notwendig kann die klare Lösung mit Impfkristallen angeimpft und danach gegebenenfalls mit Ultraschall behandelt werden. Die dabei erhaltene Mutterlauge wird vorzugsweise mit Ethanol mit einem geringen Wassergehalt verdünnt. Aus dieser verdünnten Lösung lässt man das andere Diastereoisomer auskristallisieren und filtriert es anschliessend ebenfalls ab. Diese zweite Mutterlauge kann eingeengt und einer erneuten fraktionierten Kristallisation unterworfen werden.

Welches Diastereoisomer zuerst auskristallisiert hängt unter anderem von der Natur des aufzutrennenden Ammoniumsalzes ab. Im Falle des N⁵-Methyl-THF-cyclohexylammoniumsalzes kristallisiert zuerst das (6R)-Salz aus. Das (6S)-Salz wird aus der Mutterlauge gemäss obigen Angaben auskristallisiert. Im Falle des N⁵-Methyl-THF-diisopropylammoniumsalzes kristallisiert zuerst das (6S)-Salz aus. Das (6R)-Salz wird dann aus der Mutterlauge auskristallisiert.

Die so aufgetrennten diastereoisomerenreinen Ammoniumsalze von N⁵-Methyl-THF können in die Alkalimetall- oder Erdalkalimetallsalze, insbesondere in das Calciumsalz oder in das Magnesiumsalz, übergeführt werden. Dazu wird ein entsprechendes Ammoniumsalz vorzugsweise in einem Volumenteil Wasser gelöst. Dann wird eine wässrige Lösung des gewünschten Alkalimetall- oder Erdalkalimetallsalzes hinzugegeben, beispielsweise Calciumchlorid. In diesem Gemisch kann der pH-Wert auf einen Wert von 6,0 bis 7,0 eingestellt werden. Diese Lösung wird auf eine Temperatur von 0° C bis 10° C abgekühlt und vorzugsweise etwa eine Stunde gerührt. Danach werden 1,5 bis 2,5 Volumenteile, bezogen auf obigen Volumenteil Wasser, an beispielsweise Ethanol oder Aceton hinzugegeben, vorzugsweise unter Rühren während 30 bis 90 Minuten. Dabei fällt das entsprechende Alkalimetall- oder Erdalkalimetallsalz der N⁵-Methyl-THF aus und kann abfiltriert werden.

Die Analyse einer isomeren Zusammensetzung kann routinemässig an einer chiralen HPLC Säule durchgeführt werden; insbesondere gemäss den in Referenz Nr. 12 enthaltenen Angaben.

Die nachfolgenden Beispiele dienen der Illustration der vorliegenden Erfindung.

### Beispiel 1

### Herstellung von (6RS)-N⁵-Methyl-THF-cyclohexylammoniumsalz

Zu einer Suspension von 100 g (6RS)-N⁵-Methyl-THF-calciumsalz in 800 ml Wasser, welche bei einer Temperatur von 40°C unter einer Stickstoffatmosphäre gerührt wurde, wurden 40 g Cyclohexylamin hinzugegeben. Die Temperatur wurde auf 60° C erhöht, und man erhielt eine klare Lösung. Anschliessend wurden 24 g Oxalsäuredihydrat in 200 ml Wasser hinzugegeben, und dieses Gemisch wurde bei einer Temperatur von 0°C während 30 Minuten gerührt. Nach der Entfernung des gebildeten Calciumoxalates mittels Zentrifugation wurde die überstehende Lösung bei einer Temperatur von 50°C unter reduziertem Druck eingeengt. Man erhielt rohes (6 RS)-N⁵-Methyl-THF-cyclohexylammoniumsalz in der Form eines honigartigen, resp. glasartigen Festkörpers. Die HPLC Analyse dieses Produktes zeigte einen einzigen Peak.

### Beispiel 2

### Trennung des (6RS)-Diastereoisomerengemisches von N⁵-Methyl-THF-cyclohexylammoniumsalz

### A: (6R)-N⁵-Methyl-THF-cyclohexylammoniumsalz

Das gemäss Beispiel 1 hergestellte rohe (6RS)-N⁵-Methyl-THF-cyclohexylammoniumsalz wurde in 550 ml eines Gemisches aus 85 Volumenteilen Ethanol und 15 Volumenteilen Wasser bei einer Temperatur von 60°C gelöst. Die entstandene klare Lösung wurde auf Raumtemperatur abgekühlt und mit kristallinem (6R)-N⁵-Methyl-THF-cyclohexylammoniumsalz, erhalten aus einem früheren Ansatz, angeimpft. Nach dem Rühren bei Raumtemperatur über Nacht und bei einer Temperatur von 10°C während 4 Stunden wurde der erhaltene kristalline Festkörper mittels Filtration isoliert. Dieser Festkörper wurde einmal mit 85%-igem Ethanol gewaschen und unter reduziertem Druck bei einer Temperatur von 50° C während 2 Stunden getrocknet. Man erhielt 30,9 g an (6R)-N⁵-Methyl-THF-cyclohexylammoniumsalz.
[α]_{D} = -7,3° (c=1 in 0,01 N NaOH)
Die Analyse an einer RESOLVOSIL-Säule zeigte ein Diastereoisomerenverhältnis von (6R)/(6S) = 96,5 / 3,5 (siehe die Referenz Nr. 12).

Die HPLC Analyse an einer "reverse phase"-Säule zeigte, dass dieses Produkt 100 % rein war.
IR (KBr/cm⁻¹) : 3300, 2950, 2860, 1660, 1610, 1390.

| Elementaranalyse: | berechnet (%) (Dihydrat) | gefunden (%) |
|---|---|---|
| C | 55.42 | 55.72 |
| H | 7.99 | 7.86 |
| N | 18.46 | 18.32 |

### B: (6S)-N⁵-Methyl-THF-cyclohexylammoniumsalz

Die Mutterlauge aus der in Punkt A beschriebenen Filtration wurde mit 1200 ml absolutem Ethanol verdünnt. Die erhaltene Lösung wurde mit authentischem (6S)-N⁵-Methyl-THF-cyclohexylammoniumsalz angeimpft. Man liess das Gemisch während 24 Stunden bei Raumtemperatur stehen. Der erhaltene kristalline Festkörper wurde mittels Filtration isoliert. Dieser Festkörper wurde einmal mit 94%-igem Ethanol gewaschen und unter reduziertem Druck bei einer Temperatur von 50° C getrocknet. Man erhielt 23,7 g an (6S)-N⁵-Methyl-THF-cyclohexylammoniumsalz.
[α]_{D} = +30,6° (c=1 in 0,01 N NaOH)
Die Analyse an einer RESOLVOSIL-Säule zeigte ein Diastereoisomeren-Verhältnis von (6R)/(6S) = 3/97 (siehe die Referenz Nr. 12).
Die HPLC Analyse an einer "reverse phase"-Säule zeigte, dass dieses Produkt 100 % rein war.
IR (KBr/cm⁻¹) : 3300, 2940, 2860, 1610, 1390.

| Elementaranalyse: | berechnet (%) (Dihydrat) | gefunden (%) |
|---|---|---|
| C | 55.42 | 55.72 |
| H | 7.99 | 7.86 |
| N | 18.46 | 18.32 |

### Beispiel 3

### Herstellung von (6S)-N⁵-Methyl-THF-calciumsalz

15,0 g (6S)-N⁵-Methyl-cyclohexylammoniumsalz wurden in 120 ml Wasser gelöst und bei Raumtemperatur unter einer Stickstoffatmosphäre gerührt. Nach der Hinzugabe von 9,9 ml 2M Calciumchloridlösung wurde der pH-Wert mittels der Hinzugabe von 1N NaOH auf einen Wert von 6,8 eingestellt. Das erhaltene Gemisch wurde bei einer Temperatur von 0°C während 1 Stunde gerührt. Danach wurden 240 ml Ethanol während 40 Minuten hinzugegeben. Der erhaltene kristalline Festkörper wurde mittels Filtration isoliert. Dieser Festkörper wurde mit 85%-igem Ethanol gewaschen und unter reduziertem Druck bei einer Temperatur von 50°C getrocknet. Man erhielt 10,4 g an (6S)-N⁵-Methyl-THF-calciumsalz.

Die HPLC Analyse an einer "reverse phase"-Säule zeigte, dass dieses Produkt 100 % rein war.
[α]_{D} = +40° (c=1 / 1% NH₄OAc,pH 6,0)
UV (20 mg/l in 1% NH₄OAc, pH 6,0) : λₘₐₓ = 290 nm (ε = 28600)
λₘᵢₙ = 245 nm
Aₘₐₓ/Aₘᵢₙ = 3,7
Die Analyse an einer RESOLVOSIL-Säule zeigte ein Diastereoisomerenverhältnis von (6R)/(6S) = 3/97 (siehe die Referenz Nr. 12).

### Beispiel 4

### Herstellung von (6R)-N⁵-Methyl-THF-calciumsalz

Das Titelprodukt wurde ausgehend von (6R)-N⁵-Methyl-THF-cyclohexylammoniumsalz auf analogem Wege hergestellt, wie dies im Beispiel 3 beschrieben ist.

Die HPLC Analyse an einer "reverse phase"-Säule zeigte, dass das Produkt 100 % rein war.
[α]_{D} = - 4,7° (c=1 / 1 % NH₄OAc, pH 6,0)
UV (20 mg/l in 1 % NH₄OAc, pH 6,0): λₘₐₓ = 290 nm (ε=30200)
λₘᵢₙ = 245 nm
Aₘₐₓ / Aₘᵢₙ = 3,6
Die Analyse an einer RESOLVOSIL-Säule zeigte ein Diastereoisomerenverhältnis von (6R) / (6S) = 97/3 (siehe die Referenz Nr. 12).

### Beispiel 5

### Herstellung von (6RS)-N⁵-Methyl-THF-diisopropylammoniumsalz

Zu einer Suspension von 5,0 g (6RS)-N⁵-Methyl-THF-calciumsalz in 40 ml Wasser, welche bei einer Temperatur von 40°C unter einer Stickstoffatmosphäre gerührt wurde, wurden 2,03 g Diisopropylamin hinzugegeben. Die Temperatur wurde auf etwa 55°C angehoben, und man erhielt eine klare Lösung. Nach dem Abkühlen auf Raumtemperatur wurde eine Lösung von 1,2 g Oxalsäuredihydrat in 10 ml Wasser hinzugegeben, und dieses Gemisch wurde bei einer Temperatur von 0°C während 30 Minuten gerührt. Nach der Entfernung des gebildeten Calciumoxalates mittels Zentrifugation wurde die überstehende Lösung bei einer Temperatur von 70°C unter reduziertem Druck eingeengt. Man erhielt rohes (6RS)-N⁵-Methyl-THF-diisopropylammoniumsalz in der Form eines honigartigen, resp. glasartigen Festkörpers. Die HPLC Analyse dieses Produktes zeigte einen einzigen Peak.

### Beispiel 6

### Trennung des (6RS)-Diastereoisomerengemisches von N⁵-Methyl-THF-diisopropylammoniumsalz; Isolierung von (6S)-N⁵-Methyl-THF-diisopropylammoniumsalz

Das gemäss Beispiel 5 hergestellte rohe (6RS)-N⁵-Methyl-THF-diisopropylammoniumsalz wurde in 27 ml eines Gemisches aus 85 Volumenteilen Ethanol und 15 Volumenteilen Wasser bei einer Temperatur von 60°C gelöst. Die entstandene klare Lösung wurde auf Raumtemperatur abgekühlt und bei dieser Temperatur während 48 Stunden gerührt. Der erhaltene Festkörper wurde mittels Zentrifugation isoliert. Dieser Festkörper wurde einmal mit 85%-igem Ethanol gewaschen und unter reduziertem Druck bei einer Temperatur von 50°C während 2 Stunden getrocknet. Man erhielt 1,07 g an (6S)-N⁵-Methyl-THF-diisopropylammoniumsalz.

Die Analyse an einer RESOLVOSIL-Säule zeigte ein Diastereoisomerenverhältnis von (6S)/(6R) = 90/10 (siehe die Referenz Nr. 12).

### Beispiel 7

### Herstellung von (6RS)-N⁵-Methyl-THF.2-hydroxy-ethyl-ammoniumsalz

Zu einer gerührten Suspension von 15 g (6RS)-N⁵-Methyl-THF in 100 ml 85%-igem Ethanol wurden tropfenweise 3,9 ml Ethanolamin zugegeben, wobei eine ölige Suspension erhalten wurde.

Diese Suspension wurde während 3 Stunden bei einer Temperatur von 0°C stark gerührt, und der gebildete kristalline Festkörper wurde gelegentlich mit einem Spatel zerkleinert.

Dann wurde das Produkt mittels Filtration isoliert, mit absolutem Ethanol gewaschen und unter reduziertem Druck bei einer Temperatur von 50°C während 90 Minuten getrocknet. Man erhielt 16,0 g (6RS)-N⁵-Methyl-THF.2-hydroxy-ethyl-ammoniumsalz.

Die HPLC Analyse an einer "reverse phase"-Säule zeigte, dass dieses Produkt 96,5 % rein war.

UV (20 mg/l in 1 % NH₄OAc, pH 6,0):
λₘₐₓ = 290 nm
λₘᵢₙ = 245 nm
Aₘₐₓ/Aₘᵢₙ = 3,55
IR (KBr/cm ⁻¹) : 3360, 2940, 2860, 1610, 1390, 1330.

### Beispiel 8

### Akute intravenöse Toxizitätsstudien an Mäusen

| Verbindung | LD₅₀(mg/kg) |
|---|---|
| (6RS)-N⁵-Methyl-THF.Ca²⁺ | 339 |
| (6S)-N⁵-Methyl-THF.Ca²⁺ | 847 |
| (6R)-N⁵-Methyl-THF.Ca²⁺ | 460 |

### Beispiel 9

### Inhibition des Wachstums von menschlichen CCRF-CEM Zellen

Menschliche Leukämie-Zellen (CCRF-CEM) wurden während 72 Stunden in der Gegenwart von zunehmenden Konzentrationen an (6R)-N⁵-Methyl-THF.Ca²⁺ wachsen gelassen. Die folgende Tabelle zeigt das prozentuale zelluläre Wachstum bezüglich dem Kontrollexperiment.

| Konzentration von (6R)-N⁵-Methyl-THF.Ca²⁺ [»M] | % Wachstum (72 Std. Exp.) |
|---|---|
| 0 (Kontrolle) | 100 |
| 1 | 95 |
| 10 | 99 |
| 30 | 10 |
| 100 | 9.3 |
| 250 | 8.2 |

### Referenzen

1. Cortellaro M., Boschetti C. und Polli E., High dose methotrexate with N⁵-methyl-THF rescue in acute leukemia and non-Hodgkin's lymphoma. Chemioterapia Oncologica 2 (N.4) (Dec. Suppl./1978) 311.
2. Mazzei T. et al., High dose methotrexate therapy in high-risk trophoblastic tumors. ibid. 289.
3. Novelli A. et al., Clinical data on rescue of high dose methotrexate with N⁵-methyl-THF in human solid tumors. ibid. 289.
4. Reggev A. und Djerassi I., Rescue from high-dose methotrexate with N⁵-methyl-THF. Cancer treat.Rep. (1986) 70, 251.
5. Nigra E. et al., Can folates improve the efficacy of 5-FU in a polychemotherapy schedule? A new treatment for advanced colorectal cancer. Fifth NCI-EORT Symposium on new drugs in cancer therapy, Oct. 22-24, 1986, Amsterdam (Oncology Abstracts (1987) 2, 273).
6. Chello P.L. et al., Further studies of stereospecificity at carbon 6 for membrane transport of tetrahydrofolates. Biochem. Pharmacol. (1982) 31, 1527.
7. White J.C. et al., Lack of stereospecificity at carbon 6 of N⁵-methyl-THF transport in Ehrlich ascite tumor cells. Journal of Biol. Chem. (1978) 253, 242-245.
8. White J.C. and I.D. Goldman., Lack of stereospecificity at carbon 6 of N⁵-methyl-THF transport: possible relevance to rescue regimens with methotrexate and leucovorin.
   Chemistry and Biology of Pteridines (Kisliuk/Brown eds./1979), 625.
9. B.T. Kaufman et al., Chromatographic separation of the diastereoisomers of dl,L-5,10-methylenetetrahydrofolate.
   Journal of Biol.Chem. (1963) 238, 1498.
10. "Verfahren zur Herstellung von Tetrahydrofolaten", Europäische Patentanmeldung mit der Veröffentlichungsnummer 0 348 641 (1990), Eprova AG Schaffhausen/CH.
11. "Derivés de l'acide tetrahydrofolique, procédé de préparation et utilisation dans la synthese de diasteréoisomères 6S et 6R de folates reduits" Französische Patentanmeldung Nr. 90 03032, SAPEC SA Lugano / CH.
12. K.E. Choi und R.L. Schilsky, Resolution of the stereoisomers of Leucovorin and N⁵-methyl-THF by chiral HPLC.
   Anal. Biochem. (1988) 168 , 398.
13. D.G. Weir et al., The absorption of the diastereoisomers of N⁵-methyl-THF in man: a carrier-mediated process.
   Clinical science and molecular medicine (1973) 45, 625.

## Patentansprüche (Patentansprüche für folgende(n) Vertragsstaat(en): AT, BE, CH, DE, DK, FR, GB, IT, LI, LU, NL, SE)

1. Ammoniumsalze von N⁵-Methyl-5,6,7,8-tetrahydrofolsäure der Formel I 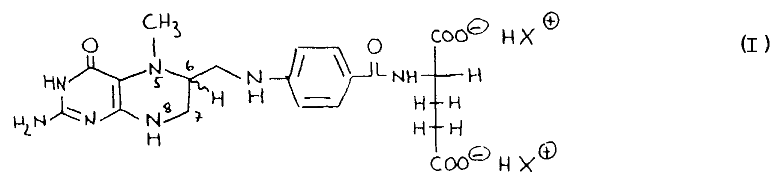 in der Form des (6RS)-Diastereoisomerengemisches oder in der Form der einzelnen (6R)-oder (6S)-Diastereoisomeren, worin
X eine Stickstoff enthaltende Base bedeutet, vorzugsweise ausgewählt aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin.

2. Verfahren zur Herstellung von Ammoniumsalzen von (6RS)-N⁵-Methyl-5,6,7,8-tetrahydrofolsäure der Formel I, welche in Anspruch 1 definiert sind,
dadurch gekennzeichnet, dass man (6RS)-N⁵-Methyl-5,6,7,8-tetrahydrofolsäure in der Form eines Alkalimetall- oder Erdalkalimetallsalzes, insbesondere Ca²⁺ oder Mg²⁺, in einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin,
dann das im ursprünglichen, oben genannten Salz enthaltene Alkalimetall- oder Erdalkalimetallkation durch Zugabe einer geeigneten Säure, zum Beispiel Schwefelsäure oder Oxalsäure, in die Form eines schwerlöslichen Salzes überführt, resp. ausfällt,
dann das so hergestellte schwerlösliche Salz abtrennt, beispielsweise mittels Filtration, und
schlussendlich das Lösungsmittel entfernt, beispielsweise mittels Verdampfung unter vermindertem Druck oder Lyophilisation.

3. Verfahren zur Herstellung von Ammoniumsalzen von (6RS)-N⁵-Methyl-5,6,7,8-tetrahydrofolsäure der Formel I, welche in Anspruch 1 definiert sind,
dadurch gekennzeichnet, dass man (6RS)-N⁵-Methyl-5,6,7,8-tetrahydrofolsäure in wenigstens einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin, und
schlussendlich das oder die Lösungsmittel entfernt, beispielsweise mittels Verdampfung unter vermindertem Druck oder Lyophilisation.

4. Verfahren zur Trennung des (6RS)-Diastereoisomerengemisches eines Ammoniumsalzes von N⁵-Methyl-5,6,7,8-tetrahydrofolsäure der Formel I, welches in Anspruch 1 definiert ist,
in die einzelnen (6R) und (6S) Diastereoisomeren,
dadurch gekennzeichnet, dass man das genannte (6RS)-Diastereoisomerengemisch, beispielsweise hergestellt gemäss dem Verfahren nach Anspruch 2 oder 3, insbesondere in fester Form, mit einem Lösungsmittelgemisch A vermischt, enthaltend Wasser und wenigstens ein mit Wasser mischbares Lösungsmittel, vorzugsweise ein polares Lösungsmittel, insbesondere ein- oder mehrwertige Alkohole mit 1 bis 5 Kohlenstoffatomen, beispielsweise Ethanol,
dann das so hergestellte Gemisch in eine klare Lösung überführt, beispielsweise mittels Erwärmung auf eine Temperatur von 40°C bis 60°C,
dann aus dieser klaren Lösung ein Diastereoisomer auskristallisieren lässt und abtrennt,
dann die Mutterlauge mit dem (den) oben erwähnten mit Wasser mischbaren Lösungsmittel(n) verdünnt, und aus dieser verdünnten Lösung das andere Diastereoisomer auskristallisieren lässt und abtrennt.

5. Verfahren nach Anspruch 4, dadurch gekennzeichnet, dass das Lösungsmittelgemisch A von 50 bis 95 Vol.-%, vorzugsweise von 75 bis 95 Vol.-%, insbesondere 85 Vol.-%, Ethanol, und als Rest Wasser enthält.

6. Verfahren nach einem der Ansprüche 4 bis 5, dadurch gekennzeichnet, dass man auf 1 Gewichtsteil (6RS)-Diastereoisomerengemisch von 3 bis 10 Volumenteile, insbesondere etwa 5 Volumenteile, Lösungsmittelgemisch A verwendet.

7. Verfahren nach einem der Ansprüche 4 bis 6, dadurch gekennzeichnet, dass man die klare Lösung mit Impfkristallen animpft, und die angeimpfte Lösung gegebenenfalls mit Ultraschall behandelt.

8. Verfahren zur Herstellung von Alkalimetall- oder Erdalkalimetallsalzen von (6R) oder (6S) N⁵-Methyl-5,6,7,8-tetrahydrofolsäure der Formeln IIa oder IIb 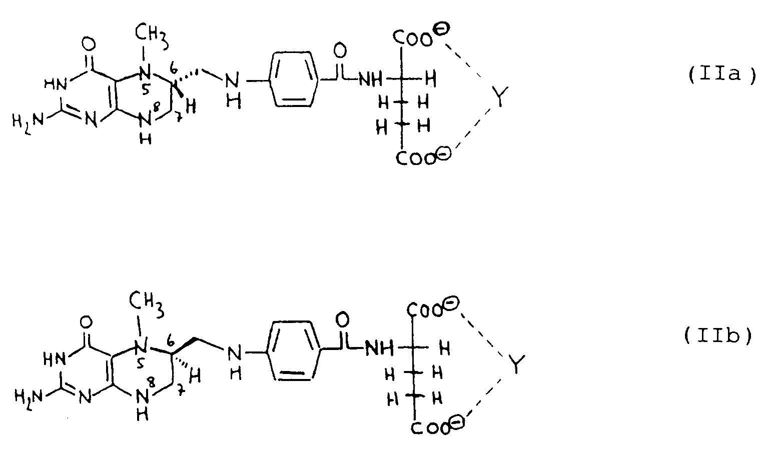 worin
Y zwei Alkalimetallkationen oder ein Erdalkalimetallkation, insbesondere Ca²⁺ oder Mg²⁺, bedeutet,
dadurch gekennzeichnet, dass man ein entsprechendes, diastereiosomerenreines Ammoniumsalz von N⁵-Methyl-5,6,7,8-tetrahydrofolsäure der Formel Ia oder Ib 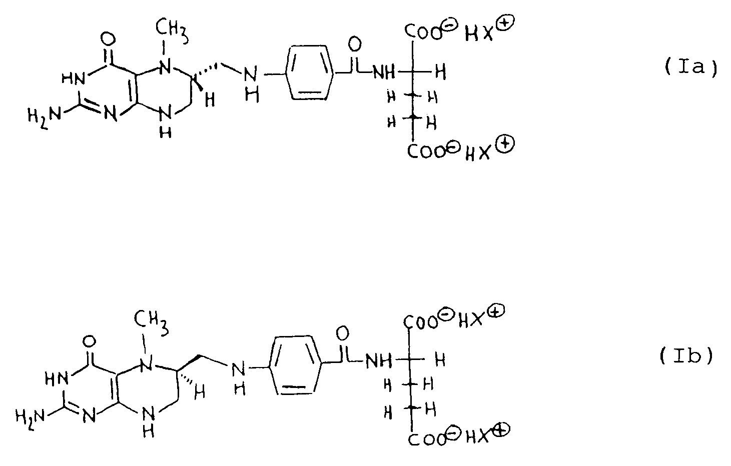 worin X in Anspruch 1 definiert ist,
beispielsweise hergestellt gemäss dem Verfahren nach einem der Ansprüche 4 bis 7,
in einem Volumenteil Wasser löst,
dann eine wässrige Lösung eines Alkalimetalloder Erdalkalimetallsalzes hinzugibt, vorzugsweise die entsprechenden Halogenide, insbesondere die entsprechenden Chloride, beispielsweise CaCl₂ oder MgCl₂,
dann das so hergestellte Gemisch B rührt, vorzugsweise bei einer Temperatur im Bereich von 0°C bis 10°C, beispielsweise während etwa 1 Stunde,
dann 1,5 bis 2,5 Volumenteile wenigstens eines polaren, mit Wasser mischbaren organischen Lösungsmittels, vorzugsweise Ethanol oder Aceton, hinzugibt, vorzugsweise unter Rühren während 30 bis 90 Minuten, und
dann das ausgefallene Alkalimetall- oder Erdalkalimetallsalz der Formel IIa oder IIb abtrennt.

9. Verfahren nach Anspruch 8, dadurch gekennzeichnet, dass man im Gemisch B den pH-Wert auf einen Wert im Bereich von 6,0 bis 7,0 einstellt.

10. Arzneimittel für die Behandlung und/oder Bekämpfung von menschlichen und/oder tierischen Tumoren und/oder für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen, dadurch gekennzeichnet, dass es als wenigstens eine aktive Verbindung wenigstens ein Ammoniumsalz von N⁵-Methyl-5,6,7,8-tetrahydrofolsäure der Formel I' 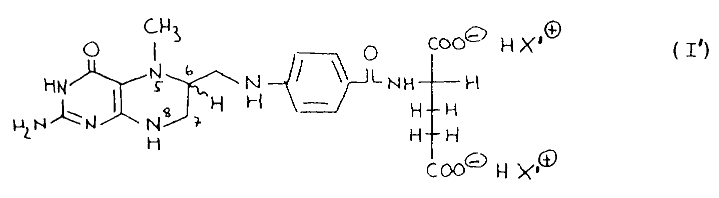 in der Form des (6RS)-Diastereoisomerengemisches oder in der Form der einzelnen (6R)-oder (6S)-Diastereoisomeren, worin
X' eine pharmakologisch annehmbare, Stickstoff enthaltende Base bedeutet, vorzugsweise ausgewählt aus primären, sekundären und tertiären Aminen sowie basischen Aminocarbonsäuren, insbesondere ausgewählt aus der Gruppe, bestehend aus Ethanolamin, Triethanolamin, 2-Dimethylamino-ethanol, tert.-Butylamin, Lysin, insbesondere (L)-Lysin, und Arginin, insbesondere (L)-Arginin,
und/oder wenigstens ein Alkalimetall- und/oder Erdalkalimetallsalz von (6R) oder (6S) N⁵-Methyl-5,6,7,8-tetrahydrofolsäure der Formeln IIa oder II b, welche in Anspruch 8 definiert sind,
enthält,
vorzugsweise in einer Menge von 1 mg bis 500 mg, insbesondere von 5 mg bis 150 mg, pro Dosiseinheit, und dass das Arzneimittel vorzugsweise in der Form eines parenteralen und/oder oralen pharmazeutischen Präparates vorliegt.

11. Verwendung der Ammoniumsalze von N⁵-Methyl-5,6,7,8-tetrahydrofolsäure der Formel I', welche in Anspruch 10 definiert sind,
und/oder der Alkalimetall- und/oder Erdalkalimetallsalze von (6R) oder (6S) N⁵-Methyl-5,6,7,8-tetrahydrofolsäure der Formeln IIa oder IIb, welche in Anspruch 8 definiert sind,
einzeln oder in beliebiger Kombination zur Herstellung eines Arzneimittels für die Behandlung und/oder Bekämpfung von menschlichen und/oder tierischen Tumoren und/oder für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen, wobei vorzugsweise diese Salze in einer Menge von 1 mg bis 500 mg, insbesondere von 5 mg bis 150 mg, pro Dosiseinheit im Arzneimittel vorhanden sind, und wobei vorzugsweise das Arzneimittel in Form eines parenteralen und/oder oralen, pharmazeutischen Präparates vorliegt.

## Patentansprüche (Patentansprüche für folgende(n) Vertragsstaat(en): ES)

1. Verfahren zur Herstellung von Ammoniumsalzen von (6RS)-N⁵-Methyl-5,6,7,8-tetrahydrofolsäure der Formel I 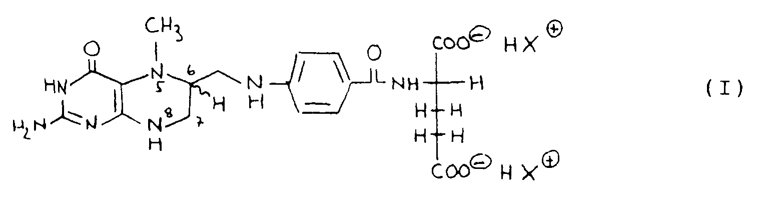 worin
X eine Stickstoff enthaltende Base bedeutet, vorzugsweise ausgewählt aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin,
dadurch gekennzeichnet, dass man (6RS)-N⁵-Methyl-5,6,7,8-tetrahydrofolsäure in der Form eines Alkalimetall- oder Erdalkalimetallsalzes, insbesondere Ca²⁺ oder Mg²⁺, in einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin,
dann das im ursprünglichen, oben genannten Salz enthaltene Alkalimetall- oder Erdalkalimetallkation durch Zugabe einer geeigneten Säure, zum Beispiel Schwefelsäure oder Oxalsäure, in die Form eines schwerlöslichen Salzes überführt, resp. ausfällt,
dann das so hergestellte schwerlösliche Salz abtrennt, beispielsweise mittels Filtration, und
schlussendlich das Lösungsmittel entfernt, beispielsweise mittels Verdampfung unter vermindertem Druck oder Lyophilisation.

2. Verfahren zur Herstellung von Ammoniumsalzen von (6RS)-N⁵-Methyl-5,6,7,8-tetrahydrofolsäure der Formel I, welche in Anspruch 1 definiert sind,
dadurch gekennzeichnet, dass man (6RS)-N⁵-Methyl-5,6,7,8-tetrahydrofolsäure in wenigstens einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin, und
schlussendlich das oder die Lösungsmittel entfernt, beispielsweise mittels Verdampfung unter vermindertem Druck oder Lyophilisation.

3. Verfahren zur Trennung des (6RS)-Diastereoisomerengemisches eines Ammoniumsalzes von N⁵-Methyl-5,6,7,8-tetrahydrofolsäure der Formel I, welches in Anspruch 1 definiert ist,
in die einzelnen (6R) und (6S) Diastereoisomeren,
dadurch gekennzeichnet, dass man das genannte (6RS)-Diastereoisomerengemisch, beispielsweise hergestellt gemäss dem Verfahren nach Anspruch 1 oder 2, insbesondere in fester Form, mit einem Lösungsmittelgemisch A vermischt, enthaltend Wasser und wenigstens ein mit Wasser mischbares Lösungsmittel, vorzugsweise ein polares Lösungsmittel, insbesondere ein- oder mehrwertige Alkohole mit 1 bis 5 Kohlenstoffatomen, beispielsweise Ethanol,
dann das so hergestellte Gemisch in eine klare Lösung überführt, beispielsweise mittels Erwärmung auf eine Temperatur von 40°C bis 60°C,
dann aus dieser klaren Lösung ein Diastereoisomer auskristallisieren lässt und abtrennt,
dann die Mutterlauge mit dem (den) oben erwähnten mit Wasser mischbaren Lösungsmittel(n) verdünnt, und aus dieser verdünnten Lösung das andere Diastereoisomer auskristallisieren lässt und abtrennt.

4. Verfahren nach Anspruch 3, dadurch gekennzeichnet, dass das Lösungsmittelgemisch A von 50 bis 95 Vol.-%, vorzugsweise von 75 bis 95 Vol.-%, insbesondere 85 Vol.-%, Ethanol, und als Rest Wasser enthält.

5. Verfahren nach einem der Ansprüche 3 bis 4, dadurch gekennzeichnet, dass man auf 1 Gewichtsteil (6RS)-Diastereoisomerengemisch von 3 bis 10 Volumenteile, insbesondere etwa 5 Volumenteile, Lösungsmittelgemisch A verwendet.

6. Verfahren nach einem der Ansprüche 3 bis 5, dadurch gekennzeichnet, dass man die klare Lösung mit Impfkristallen animpft, und die angeimpfte Lösung gegebenenfalls mit Ultraschall behandelt.

7. Verfahren zur Herstellung von Alkalimetall- oder Erdalkalimetallsalzen von (6R) oder (6S) N⁵-Methyl-5,6,7,8-tetrahydrofolsäure der Formeln IIa oder IIb 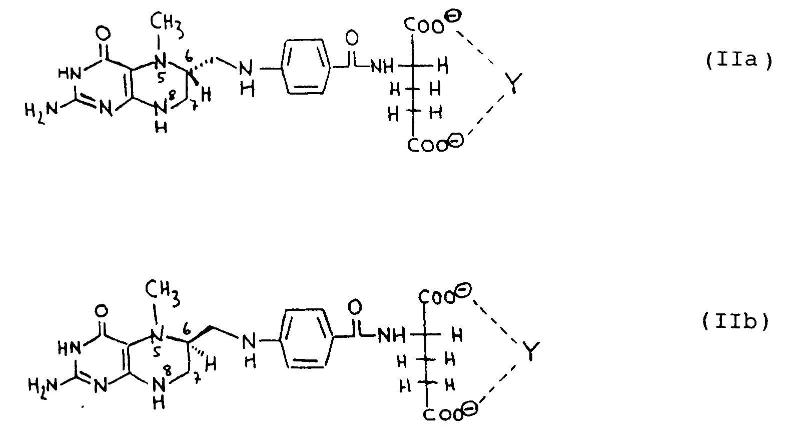 worin
Y zwei Alkalimetallkationen oder ein Erdalkalimetallkation, insbesondere Ca²⁺ oder Mg²⁺, bedeutet,
dadurch gekennzeichnet, dass man ein entsprechendes, diastereiosomerenreines Ammoniumsalz von N⁵-Methyl-5,6,7,8-tetrahydrofolsäure der Formel Ia oder Ib 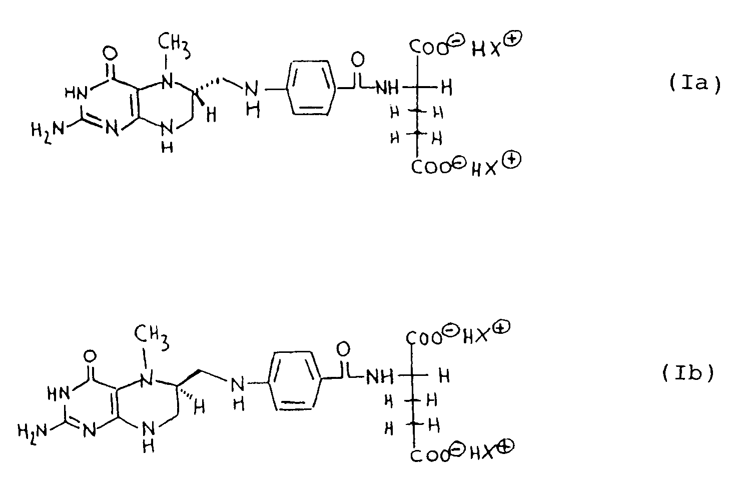 worin X in Anspruch 1 definiert ist,
beispielsweise hergestellt gemäss dem Verfahren nach einem der Ansprüche 3 bis 6,
in einem Volumenteil Wasser löst,
dann eine wässrige Lösung eines Alkalimetall- oder Erdalkalimetallsalzes hinzugibt, vorzugsweise die entsprechenden Halogenide, insbesondere die entsprechenden Chloride, beispielsweise CaCl₂ oder MgCl₂,
dann das so hergestellte Gemisch B rührt, vorzugsweise bei einer Temperatur im Bereich von 0°C bis 10°C, beispielsweise während etwa 1 Stunde,
dann 1,5 bis 2,5 Volumenteile wenigstens eines polaren, mit Wasser mischbaren organischen Lösungsmittels, vorzugsweise Ethanol oder Aceton, hinzugibt, vorzugsweise unter Rühren während 30 bis 90 Minuten, und
dann das ausgefallene Alkalimetall- oder Erdalkalimetallsalz der Formel IIa oder IIb abtrennt.

8. Verfahren nach Anspruch 7, dadurch gekennzeichnet, dass man im Gemisch B den pH-Wert auf einen Wert im Bereich von 6,0 bis 7,0 einstellt.

9. Verfahren zur Herstellung von Arzneimitteln für die Behandlung und/oder Bekämpfung von menschlichen und/oder tierischen Tumoren und/oder für die synergistische Beeinflussung einer Krebs bekämpfenden Verbindung und/oder für die Verminderung der Toxizität einer Krebs bekämpfenden Verbindung und/oder zum Schutz menschlicher und/oder tierischen Zellen, dadurch gekennzeichnet, dass man im Arzneimittel, nebst üblichen Hilfs- und Trägerstoffen, als wenigstens eine aktive Verbindung, wenigstens ein Salz gemäss den vorhergehenden Ansprüchen einmischt, vorzugsweise in einer Menge von 1 mg bis 500 mg, insbesondere 5 mg bis 150 mg, pro Dosiseinheit, wobei das Arzneimittel in Form eines parenteralen und/oder oralen pharmazeutischen Präparates vorliegt, und als solches verabreicht wird.

## Patentansprüche (Patentansprüche für folgende(n) Vertragsstaat(en): GR)

1. Ammoniumsalze von N⁵-Methyl-5,6,7,8-tetrahydrofolsäure der Formel I 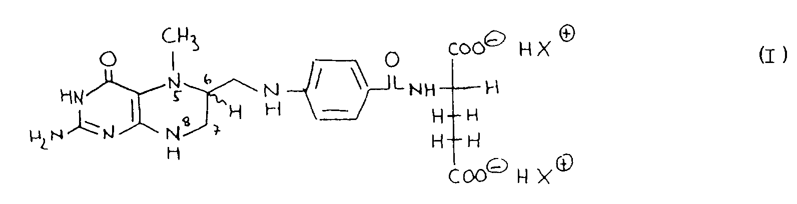 in der Form des (6RS)-Diastereoisomerengemisches oder in der Form der einzelnen (6R)-oder (6S)-Diastereoisomeren, worin
X eine Stickstoff enthaltende Base bedeutet, vorzugsweise ausgewählt aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin, die nach Anspruch 2 oder 3 hergestellt werden.

2. Verfahren zur Herstellung von Ammoniumsalzen von (6RS)-N⁵-Methyl-5,6,7,8-tetrahydrofolsäure der Formel I, welche in Anspruch 1 definiert sind,
dadurch gekennzeichnet, dass man (6RS)-N⁵-Methyl-5,6,7,8-tetrahydrofolsäure in der Form eines Alkalimetall- oder Erdalkalimetallsalzes, insbesondere Ca²⁺ oder Mg²⁺, in einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin,
dann das im ursprünglichen, oben genannten Salz enthaltene Alkalimetall- oder Erdalkalimetallkation durch Zugabe einer geeigneten Säure, zum Beispiel Schwefelsäure oder Oxalsäure, in die Form eines schwerlöslichen Salzes überführt, resp. ausfällt,
dann das so hergestellte schwerlösliche Salz abtrennt, beispielsweise mittels Filtration, und
schlussendlich das Lösungsmittel entfernt, beispielsweise mittels Verdampfung unter vermindertem Druck oder Lyophilisation.

3. Verfahren zur Herstellung von Ammoniumsalzen von (6RS)-N⁵-Methyl-5,6,7,8-tetrahydrofolsäure der Formel I, welche in Anspruch 1 definiert sind,
dadurch gekennzeichnet, dass man (6RS)-N⁵-Methyl-5,6,7,8-tetrahydrofolsäure in wenigstens einem Lösungsmittel, vorzugsweise Wasser, vorlegt,
dann eine Stickstoff enthaltende Base hinzugibt, vorzugsweise in einer Menge von 2 bis 2,5 Moläquivalente, wobei diese Base vorzugsweise ausgewählt ist aus primären, sekundären und tertiären Aminen sowie basischen Aminocarbonsäuren, insbesondere ausgewählt aus der Gruppe, bestehend aus Cyclohexylamin, Diisopropylamin, Benzylamin, Ammoniak, Ethanolamin, Triethanolamin, 2-Dimethylamino-ethanol, tert.-Butylamin, Lysin, insbesondere (L)-Lysin, und Arginin, insbesondere (L)-Arginin, und
schlussendlich das oder die Lösungsmittel entfernt, beispielsweise mittels Verdampfung unter vermindertem Druck oder Lyophilisation.

4. Verfahren zur Trennung des (6RS)-Diastereoisomerengemisches eines Ammoniumsalzes von N⁵-Methyl-5,6,7,8-tetrahydrofolsäure der Formel I, welches in Anspruch 1 definiert ist,
in die einzelnen (6R) und (6S) Diastereoisomeren,
dadurch gekennzeichnet, dass man das genannte (6RS)-Diastereoisomerengemisch, beispielsweise hergestellt gemäss dem Verfahren nach Anspruch 2 oder 3, insbesondere in fester Form, mit einem Lösungsmittelgemisch A vermischt, enthaltend Wasser und wenigstens ein mit Wasser mischbares Lösungsmittel, vorzugsweise ein polares Lösungsmittel, insbesondere ein- oder mehrwertige Alkohole mit 1 bis 5 Kohlenstoffatomen, beispielsweise Ethanol,
dann das so hergestellte Gemisch in eine klare Lösung überführt, beispielsweise mittels Erwärmung auf eine Temperatur von 40°C bis 60°C,
dann aus dieser klaren Lösung ein Diastereoisomer auskristallisieren lässt und abtrennt,
dann die Mutterlauge mit dem (den) oben erwähnten mit Wasser mischbaren Lösungsmittel(n) verdünnt, und aus dieser verdünnten Lösung das andere Diastereoisomer auskristallisieren lässt und abtrennt.

5. Verfahren nach Anspruch 4, dadurch gekennzeichnet, dass das Lösungsmittelgemisch A von 50 bis 95 Vol.-%, vorzugsweise von 75 bis 95 Vol.-%, insbesondere 85 Vol.-%, Ethanol, und als Rest Wasser enthält.

6. Verfahren nach einem der Ansprüche 4 bis 5, dadurch gekennzeichnet, dass man auf 1 Gewichtsteil (6RS)-Diastereoisomerengemisch von 3 bis 10 Volumenteile, insbesondere etwa 5 Volumenteile, Lösungsmittelgemisch A verwendet.

7. Verfahren nach einem der Ansprüche 4 bis 6, dadurch gekennzeichnet, dass man die klare Lösung mit Impfkristallen animpft, und die angeimpfte Lösung gegebenenfalls mit Ultraschall behandelt.

8. Verfahren zur Herstellung von Alkalimetall- oder Erdalkalimetallsalzen von (6R) oder (6S) N⁵-Methyl-5,6,7,8-tetrahydrofolsäure der Formeln IIa oder IIb 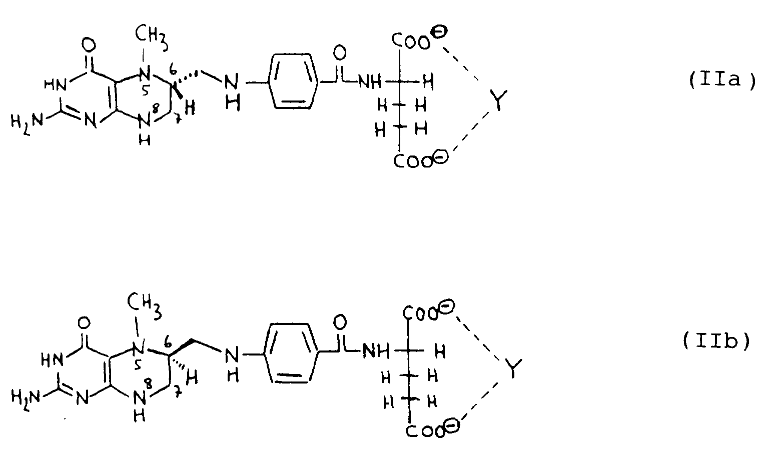 worin
Y zwei Alkalimetallkationen oder ein Erdalkalimetallkation, insbesondere Ca²⁺ oder Mg²⁺, bedeutet,
dadurch gekennzeichnet, dass man ein entsprechendes, diastereiosomerenreines Ammoniumsalz von N⁵-Methyl-5,6,7,8-tetrahydrofolsäure der Formel Ia oder Ib 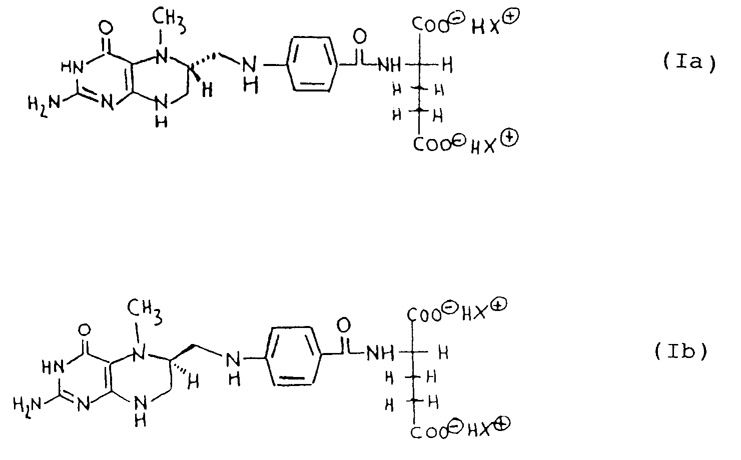 worin X in Anspruch 1 definiert ist,
beispielsweise hergestellt gemäss dem Verfahren nach einem der Ansprüche 4 bis 7,
in einem Volumenteil Wasser löst,
dann eine wässrige Lösung eines Alkalimetall- oder oder Erdalkalimetallsalzes hinzugibt, vorzugsweise die entsprechenden Halogenide, insbesondere die entsprechenden Chloride, beispielsweise CaCl₂ oder MgCl₂,
dann das so hergestellte Gemisch B rührt, vorzugsweise bei einer Temperatur im Bereich von 0°C bis 10°C, beispielsweise während etwa 1 Stunde,
dann 1,5 bis 2,5 Volumenteile wenigstens eines polaren, mit Wasser mischbaren organischen Lösungsmittels, vorzugsweise Ethanol oder Aceton, hinzugibt, vorzugsweise unter Rühren während 30 bis 90 Minuten, und
dann das ausgefallene Alkalimetall- oder Erdalkalimetallsalz der Formel IIa oder IIb abtrennt.

9. Verfahren nach Anspruch 8, dadurch gekennzeichnet, dass man im Gemisch B den pH-Wert auf einen Wert im Bereich von 6,0 bis 7,0 einstellt.

10. Verwendung der Ammoniumsalze von N⁵-Methyl-5,6,7,8-tetrahydrofolsäure der Formel I' 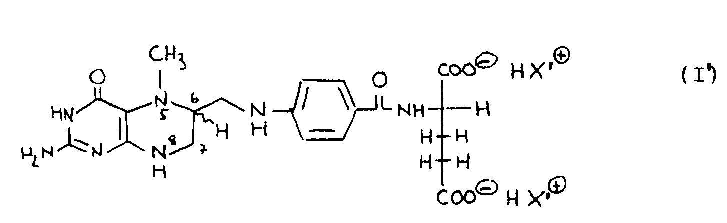 in der Form des (6RS)-Diastereoisomerengemisches oder in der Form der einzelnen (6R)- oder (6S)-Diastereoisomeren, worin
X' eine pharmakologisch annehmbare, Stickstoff enthaltende Base bedeutet, vorzugsweise ausgewählt aus primären, sekundären und tertiären Aminen sowie basischen Aminocarbonsäuren, insbesondere ausgewählt aus der Gruppe, bestehend aus Ethanolamin, Triethanolamin, 2-Dimethylamino-ethanol, tert.-Butylamin, Lysin, insbesondere (L)-Lysin, und Arginin, insbesondere (L)-Arginin, die nach Anspruch 2 oder 3 hergestellt werden,
und/oder der Alkalimetall- und/oder Erdalkalimetallsalze von (6R) oder (6S) N⁵-Methyl-5,6,7,8-tetrahydrofolsäure der Formeln IIa oder IIb, welche in Anspruch 8 definiert sind,
die nach Anspruch 8 hergestellt werden,
einzeln oder in beliebiger Kombination zur Herstellung eines Arzneimittels für die Behandlung und/oder Bekämpfung von menschlichen und/oder tierischen Tumoren und/oder für die synergistische Beeinflussung einer Krebs bekämpfenden Verbindung und/oder für die Verminderung der Toxizität einer Krebs bekämpfenden Verbindung und/oder zum Schutz menschlicher und/oder tierischer Zellen, wobei vorzugsweise diese Salze in einer Menge von 1 mg bis 500 mg, insbesondere von 5 mg bis 150 mg, pro Dosiseinheit im Arzneimittel vorhanden sind, und wobei vorzugsweise das Arzneimittel in Form eines parenteralen und/oder oralen, pharmazeutischen Präparates vorliegt.

## Claims (Claims for the following Contracting State(s): AT, BE, CH, DE, DK, FR, GB, IT, LI, LU, NL, SE)

1. Ammonium salts of N⁵-methyl-5,6,7,8-tetrahydrofolic acid of the formula I 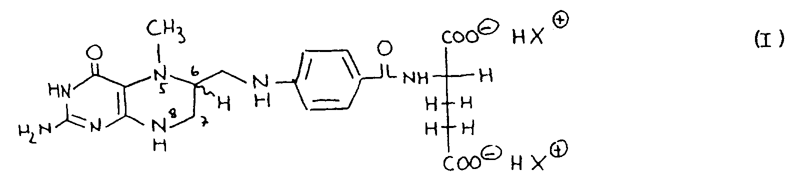 in the form of the mixture of the (6RS)-diastereoisomers or in the form of the single (6R)-or (6S)-diastereoisomers, wherein
X is a nitrogen containing base, preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine.

2. A process for the preparation of ammonium salts of (6RS)-N⁵-methyl-5,6,7,8-tetrahydrofolic acid of the formula I, which are defined in claim 1,
characterized in that (6RS)-N⁵-methyl-5,6,7,8-tetrahydrofolic acid in the form of an alkali metal or alkaline earth metal salt, especially Ca²⁺ or Mg²⁺, is placed into a solvent, preferably water,
then a nitrogen containing base is added, preferably in an amount from 2 to 2,5 molequivalents, whereby this base is preferably selected of from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine,
then transforming in the form of a difficultly soluble salt or precipitating, respectively the in the original, above mentioned salt contained alkali metal or alkaline earth metal cation by the addition of a suitable acid, for example sulfuric acid or oxalic acid,
then separating the so prepared difficultly soluble salt, for example by means of filtration, and
finally removing the solvent, for example by means of evaporation under reduced pressure or by means of lyophilization.

3. A process for the preparation of ammonium salts of (6RS)-N⁵-methyl-5,6,7,8-tetrahydrofolic acid of the formula I, which are defined in claim 1,
characterized in that (6RS)-N⁵-methyl-5,6,7,8-tetrahydrofolic acid is placed into at least one solvent, preferably water,
then adding a nitrogen containing base, preferably in an amount from 2 to 2,5 molequivalents, whereby this base is preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine, and
finally removing the solvent(s), for example by means of evaporation under reduced pressure or by means of lyophilization.

4. A process for the separation of the mixture of (6RS)-diastereoisomers of an ammonium salt of N⁵-methyl-5,6,7,8-tetrahydrofolic acid of the formula I, which is defined in claim 1,
into the single (6R) and (6S)-diastereoisomers,
characterized in that said mixture of (6RS)-diastereoisomers, for example prepared according to the process of claim 2 or 3, especially in solid form, is mixed with a mixture A of solvents, containing water and at least one with water miscible solvent, preferably a polar solvent, especially monovalent or polyhydric alcohols with 1 to 5 carbon atoms, for example ethanol,
then transforming the so prepared mixture into a clear solution, for example by means of heating to a temperature from 40°C to 60°C,
then allowing to crystallize and separating from this clear solution one diastereoisomer,
then diluting the mother liquor with the above mentioned with water miscible solvent(s), and allowing to crystallize and separating from this diluted solution the other diastereoisomer.

5. The process according to claim 4, characterized in that the mixture A of solvents contains from 50 to 95 vol.-%, preferably from 75 to 95 vol.-%, especially 85 vol.-%, of ethanol and as residue water.

6. The process according to one of claims 4 to 5, characterized in that per 1 part by weight of the mixture of (6RS)-diastereoisomers from 3 to 10 parts by volume, especially about 5 parts by volume, of the mixture A of solvents are used.

7. The process according to one of claims 4 to 6, characterized in that the clear solution is treated with seed crystals, and in that the solution, treated with seed crystals, is occasionally treated with ultra sonics.

8. A process for the preparation of alkali metal or alkaline earth metal salts of (6R) or (6S)-N⁵-methyl-5,6,7,8-tetrahydrofolic acid of the formulae IIa or IIb 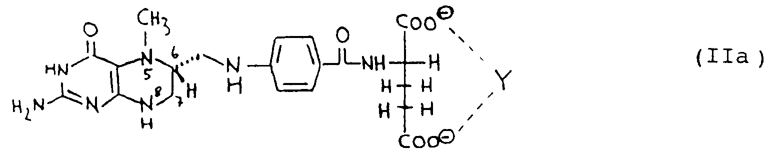 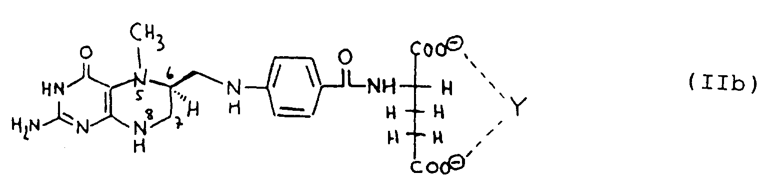 wherein
Y is defined as two alkali metal cations or as one alkaline earth metal cation, especially Ca²⁺ or Mg²⁺,
characterized in that a corresponding diastereoisomeric pure ammonium salt of N⁵-methyl-5,6,7,8-tetrahydrofolic acid of the formulae Ia or Ib 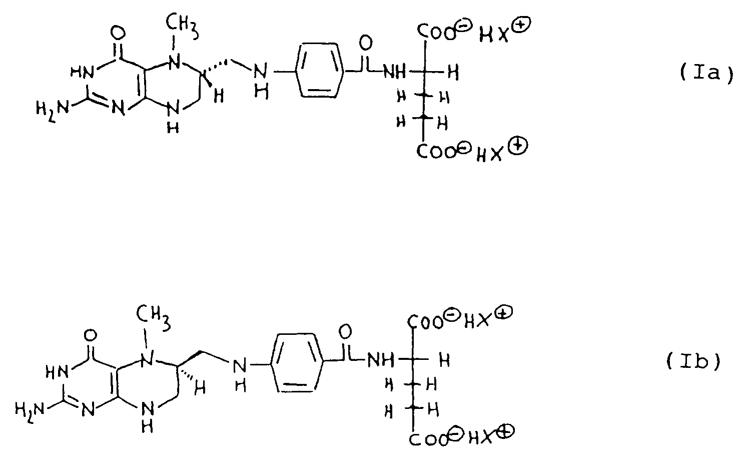 wherein X is defined in claim 1,
for example prepared according to the process according to one of claims 4 to 7,
is dissolved in one part by volume of water,
then is added an aqueous solution of an alkali metal or alkaline earth metal salt, preferably the corresponding halides, especially the corresponding chlorides, for example CaCl₂ or MgCl₂,
then stirring the so prepared mixture B, preferably at a temperature in the range from 0°C to 10°C, for example during about one hour,
then adding 1,5 to 2,5 parts by volume of at least one polar, with water miscible organic solvent, preferably ethanol or acetone, preferably under stirring during 30 to 90 minutes, and
then separating the precipitated alkali metal or alkaline earth metal salt of the formula IIa or IIb.

9. The process according to claim 8, characterized in that in the mixture B the pH-value is adjusted to a value in the range from 6,0 to 7,0.

10. A medicament for the treatment and/or the control of human and/or animal tumors and/or for the synergistic exertion of influence of a cancer controlling compound and/or for the reduction of the toxicity of a cancer controlling compound and/or for the protection of human and/or animal cells, characterized in that it contains as at least one active compound at least one ammonium salt of N⁵-methyl-5,6,7,8-tetrahydrofolic acid of the formula I' 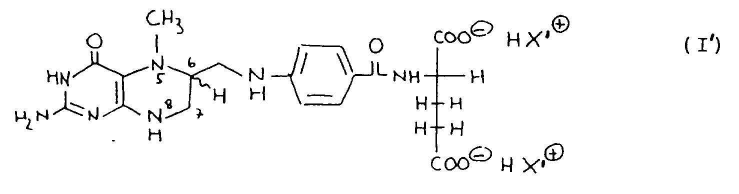 in the form of the mixture of the (6RS)-diastereoisomers or in the form of the single (6R)-or (6S)-diastereoisomers,
wherein
X' is a pharmacological acceptable, nitrogen containing base, preferably selected from primary, secondary and tertiary amines as well as basic amino carboxylic acids, especially selected from the group consisting of ethanolamine, triethanolamine, 2-dimethylaminoethanol, tert.-butylamine, lysine, especially (L)-lysine, and arginine, especially (L)-arginine,
and/or at least one alkali metal and/or alkaline earth metal salt of (6R) or (6S) N⁵-methyl-5,6,7,8-tetrahydrofolic acid of the formulae IIa or IIb, which are defined in claim 8,
preferably in an amount from 1 mg to 500 mg, especially from 5 mg to 150 mg, per dosis unit, and that the medicament is preferably in the form of a parenteral and/or oral pharmaceutical preparation.

11. Use of the ammonium salts of N⁵-methyl-5,6,7,8-tetrahydrofolic acid of the formula I', which are defined in claim 10,
and/or of the alkali metal and/or alkaline earth metal salts of (6R) or (6S) N⁵-methyl-5,6,7,8-tetrahydrofolic acid of the formulae IIa or IIb, which are defined in claim 8,
in a single form or in any combination for the preparation of a medicament for the treatment and/or the control of human and/or animal tumors and/or for the synergistic exertion of influence of a cancer controlling compound and/or for the reduction of the toxicity of a cancer controlling compound and/or the protection of human and/or animal cells, whereby these salts are preferably present in the medicament in an amount from 1 mg to 500 mg, especially from 5 mg to 150 mg, per dosis unit, and whereby the medicament is preferably in the form of a parenteral and/or oral, pharmaceutical preparation.

## Claims (Claims for the following Contracting State(s): ES)

1. A process for the preparation of ammonium salts of (6RS)-N⁵-methyl-5,6,7,8-tetrahydrofolic acid of the formula I 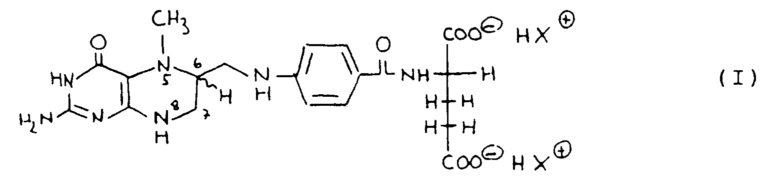 wherein
X is a nitrogen containing base, preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine,
characterized in that (6RS)-N⁵-methyl-5,6,7,8-tetrahydrofolic acid in the form of an alkali metal or alkaline earth metal salt, especially Ca²⁺ or Mg²⁺, is placed into a solvent, preferably water,
then a nitrogen containing base is added, preferably in an amount from 2 to 2,5 molequivalents, whereby this base is preferably selected of from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine,
then transforming in the form of a difficultly soluble salt or precipitating, respectively the in the original, above mentioned salt contained alkali metal or alkaline earth metal cation by the addition of a suitable acid, for example sulfuric acid or oxalic acid,
then separating the so prepared difficultly soluble salt, for example by means of filtration, and
finally removing the solvent, for example by means of evaporation under reduced pressure or by means of lyophilization.

2. A process for the preparation of ammonium salts of (6RS)-N⁵-methyl-5,6,7,8-tetrahydrofolic acid of the formula I, which are defined in claim 1,
characterized in that (6RS)-N⁵-methyl-5,6,7,8-tetrahydrofolic acid is placed into at least one solvent, preferably water,
then adding a nitrogen containing base, preferably in an amount from 2 to 2,5 molequivalents, whereby this base is preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine, and
finally removing the solvent(s), for example by means of evaporation under reduced pressure or by means of lyophilization.

3. A process for the separation of the mixture of (6RS)-diastereoisomers of an ammonium salt of N⁵-methyl-5,6,7,8-tetrahydrofolic acid of the formula I, which is defined in claim 1,
into the single (6R) and (6S)-diastereoisomers,
characterized in that said mixture of (6RS)-diastereoisomers, for example prepared according to the process of claim 1 or 2, especially in solid form, is mixed with a mixture A of solvents, containing water and at least one with water miscible solvent, preferably a polar solvent, especially monovalent or polyhydric alcohols with 1 to 5 carbon atoms, for example ethanol,
then transforming the so prepared mixture into a clear solution, for example by means of heating to a temperature from 40°C to 60°C,
then allowing to crystallize and separating from this clear solution one diastereoisomer,
then diluting the mother liquor with the above mentioned with water miscible solvent(s), and allowing to crystallize and separating from this diluted solution the other diastereoisomer.

4. The process according to claim 3, characterized in that the mixture A of solvents contains from 50 to 95 vol.-%, preferably from 75 to 95 vol.-%, especially 85 vol.-%, of ethanol and as residue water.

5. The process according to one of claims 3 to 4, characterized in that per 1 part by weight of the mixture of (6RS)-diastereoisomers from 3 to 10 parts by volume, especially about 5 parts by volume, of the mixture A of solvents are used.

6. The process according to one of claims 3 to 5, characterized in that the clear solution is treated with seed crystals, and in that the solution, treated with seed crystals, is occasionally treated with ultra sonics.

7. A process for the preparation of alkali metal or alkaline earth metal salts of (6R) or (6S)-N⁵-methyl-5,6,7,8-tetrahydrofolic acid of the formulae IIa or IIb 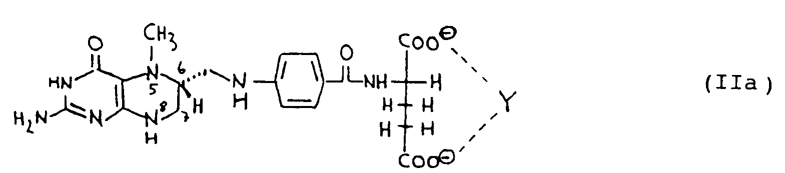 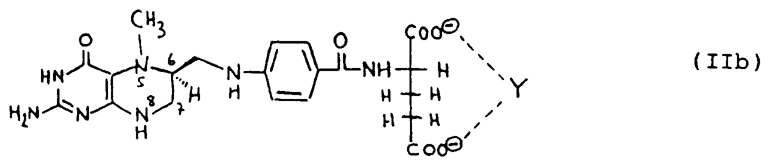 wherein
Y is defined as two alkali metal cations or as one alkaline earth metal cation, especially Ca²⁺ or Mg²⁺,
characterized in that a corresponding diastereoisomeric pure ammonium salt of N⁵-methyl-5,6,7,8-tetrahydrofolic acid of the formulae Ia or Ib 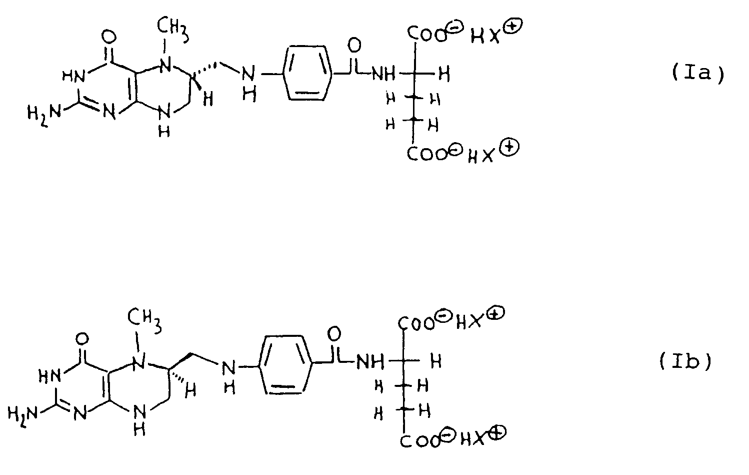 wherein X is defined in claim 1,
for example prepared according to the process according to one of claims 3 to 6,
is dissolved in one part by volume of water,
then is added an aqueous solution of an alkali metal or alkaline earth metal salt, preferably the corresponding halides, especially the corresponding chlorides, for example CaCl₂ or MgCl₂,
then stirring the so prepared mixture B, preferably at a temperature in the range from 0°C to 10°C, for example during about one hour,
then adding 1,5 to 2,5 parts by volume of at least one polar, with water miscible organic solvent, preferably ethanol or acetone, preferably under stirring during 30 to 90 minutes, and
then separating the precipitated alkali metal or alkaline earth metal salt of the formula IIa or IIb.

8. The process according to claim 7, characterized in that in the mixture B the pH-value is adjusted to a value in the range from 6,0 to 7,0.

9. A process for the preparation of a medicament for the treatment and/or the control of human and/or animal tumors and/or for the synergistic exertion of influence of a cancer controlling compound and/or for the reduction of the toxicity of a cancer controlling compound and/or for the protection of human and/or animal cells, characterized in that into the medicament is mixed, beside usual adjuvants and carrier substances, as at least one active compound, at least one salt according to the proceeding claims, preferably in an amount from 1 mg to 500 mg, especially from 5 mg to 150 mg, per dosis unit, whereby the medicament is in the form of a parenteral and/or oral pharmaceutical preparation, and is administered as such.

## Claims (Claims for the following Contracting State(s): GR)

1. Ammonium salts of N⁵-methyl-5,6,7,8-tetrahydrofolic acid of the formula I 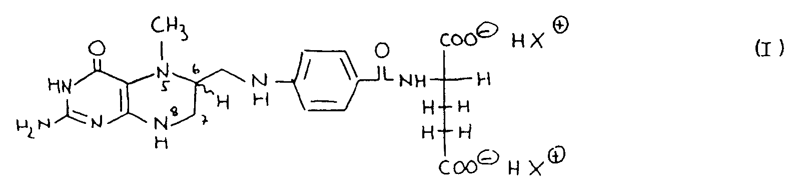 in the form of the mixture of the (6RS)-diastereoisomers or in the form of the single (6R)-or (6S)-diastereoisomers, wherein
X is a nitrogen containing base, preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine, which are prepared according to claim 2 or 3.

2. A process for the preparation of ammonium salts of (6RS)-N⁵-methyl-5,6,7,8-tetrahydrofolic acid of the formula I, which are defined in claim 1,
characterized in that (6RS)-N⁵-methyl-5,6,7,8-tetrahydrofolic acid in the form of an alkali metal or alkaline earth metal salt, especially Ca²⁺ or Mg²⁺, is placed into a solvent, preferably water,
then a nitrogen containing base is added, preferably in an amount from 2 to 2,5 molequivalents, whereby this base is preferably selected of from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine,
then transforming in the form of a difficultly soluble salt or precipitating, respectively the in the original, above mentioned salt contained alkali metal or alkaline earth metal cation by the addition of a suitable acid, for example sulfuric acid or oxalic acid,
then separating the so prepared difficultly soluble salt, for example by means of filtration, and
finally removing the solvent, for example by means of evaporation under reduced pressure or by means of lyophilization.

3. A process for the preparation of ammonium salts of (6RS)-N⁵-methyl-5,6,7,8-tetrahydrofolic acid of the formula I, which are defined in claim 1,
characterized in that (6RS)-N⁵-methyl-5,6,7,8-tetrahydrofolic acid is placed into at least one solvent, preferably water,
then adding a nitrogen containing base, preferably in an amount from 2 to 2,5 molequivalents, whereby this base is preferably selected from primary, secondary and tertiary amines as well as basic amino carboxylic acids, especially selected from the group, consisting of cyclohexyl amine, diisopropyl amine, benzyl amine, ammonia, ethanol amine, triethanol amine, 2-dimethyl amino-ethanol, tert.-butyl amine, lysine, especially (L)-lysine, and arginine, especially (L)-arginine, and
finally removing the solvent(s), for example by means of evaporation under reduced pressure or by means of lyophilization.

4. A process for the separation of the mixture of (6RS)-diastereoisomers of an ammonium salt of N⁵-methyl-5,6,7,8-tetrahydrofolic acid of the formula I, which is defined in claim 1,
into the single (6R) and (6S)-diastereoisomers,
characterized in that said mixture of (6RS)-diastereoisomers, for example prepared according to the process of claim 2 or 3, especially in solid form, is mixed with a mixture A of solvents, containing water and at least one with water miscible solvent, preferably a polar solvent, especially monovalent or polyhydric alcohols with 1 to 5 carbon atoms, for example ethanol,
then transforming the so prepared mixture into a clear solution, for example by means of heating to a temperature from 40°C to 60°C,
then allowing to crystallize and separating from this clear solution one diastereoisomer,
then diluting the mother liquor with the above mentioned with water miscible solvent(s), and allowing to crystallize and separating from this diluted solution the other diastereoisomer.

5. The process according to claim 4, characterized in that the mixture A of solvents contains from 50 to 95 vol.-%, preferably from 75 to 95 vol.-%, especially 85 vol.-%, of ethanol and as residue water.

6. The process according to one of claims 4 to 5, characterized in that per 1 part by weight of the mixture of (6RS)-diastereoisomers from 3 to 10 parts by volume, especially about 5 parts by volume, of the mixture A of solvents are used.

7. The process according to one of claims 4 to 6, characterized in that the clear solution is treated with seed crystals, and in that the solution, treated with seed crystals, is occasionally treated with ultra sonics.

8. A process for the preparation of alkali metal or alkaline earth metal salts of (6R) or (6S)-N⁵-methyl-5,6,7,8-tetrahydrofolic acid of the formulae IIa or IIb 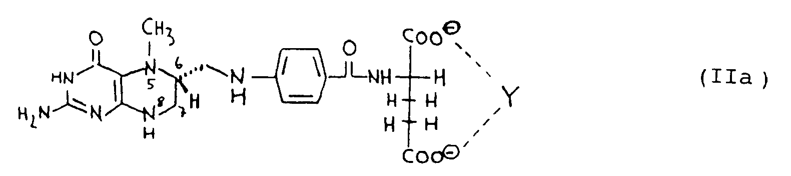 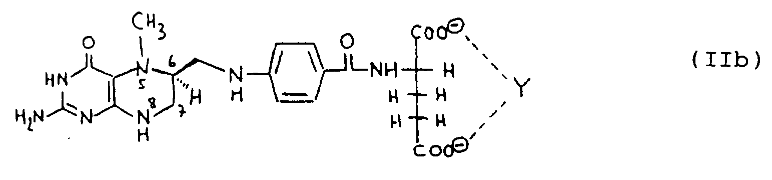 wherein
Y is defined as two alkali metal cations or as one alkaline earth metal cation, especially Ca²⁺ or Mg²⁺,
characterized in that a corresponding diastereoisomeric pure ammonium salt of N⁵-methyl-5,6,7,8-tetrahydrofolic acid of the formulae Ia or Ib 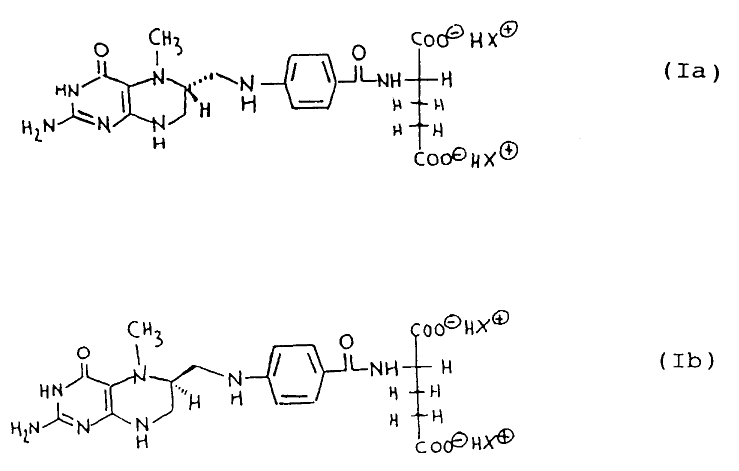 wherein X is defined in claim 1,
for example prepared according to the process according to one of claims 4 to 7,
is dissolved in one part by volume of water,
then is added an aqueous solution of an alkali metal or alkaline earth metal salt, preferably the corresponding halides, especially the corresponding chlorides, for example CaCl₂ or MgCl₂,
then stirring the so prepared mixture B, preferably at a temperature in the range from 0°C to 10°C, for example during about one hour,
then adding 1,5 to 2,5 parts by volume of at least one polar, with water miscible organic solvent, preferably ethanol or acetone, preferably under stirring during 30 to 90 minutes, and
then separating the precipitated alkali metal or alkaline earth metal salt of the formula IIa or IIb.

9. The process according to claim 8, characterized in that in the mixture B the pH-value is adjusted to a value in the range from 6,0 to 7,0.

10. Use of the ammonium salts of N⁵-methyl-5,6,7,8-tetrahydrofolic acid of the formula I' 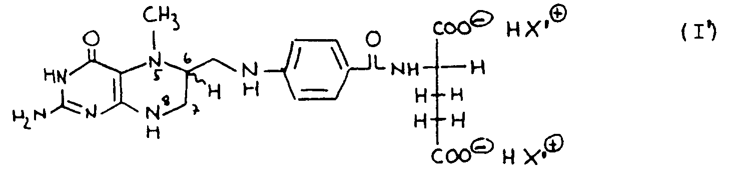 in the form of the mixture of the (6RS)-diastereoisomers or in the form of the single (6R)- or (6S)-diastereoisomers, wherein
X' is a pharmacological acceptable, nitrogen containing base, preferably selected from primary, secondary and tertiary amines as well as basic amino carboxylic acids, especially selected from the group, consisting of ethanolamine, triethanolamine, 2-dimethylamino-ethanol, tert.-butylamine, lysine, especially (L)-lysine, and arginine, especially (L)-arginine, which are prepared according to claim 2 or 3,
and/or of the alkali metal and/or alkaline earth metal salts of (6R) or (6S) N⁵-methyl-5,6,7,8-tetrahydrofolic acid of the formulae IIa or IIb, which are defined in claim 8, and which are prepared according to claim 8,
in a single form or in any combination for the preparation of a medicament for the treatment and/or the control of human and/or animal tumors and/or for the synergistic exertion of influence of a cancer controlling compound and/or for the reduction of the toxicity of a cancer controlling compound and/or the protection of human and/or animal cells, whereby these salts are preferably present in the medicament in an amount from 1 mg to 500 mg, especially from 5 mg to 150 mg, per dosis unit, and whereby the medicament is preferably in the form of a parenteral and/or oral, pharmaceutical preparation.

## Revendications (Revendications pour l'(les) Etat(s) contractant(s) suivant(s): AT, BE, CH, DE, DK, FR, GB, IT, LI, LU, NL, SE)

1. Sels d'ammonium de l'acide N⁵-méthyl-5,6,7,8-tétrahydrofolique de formule I 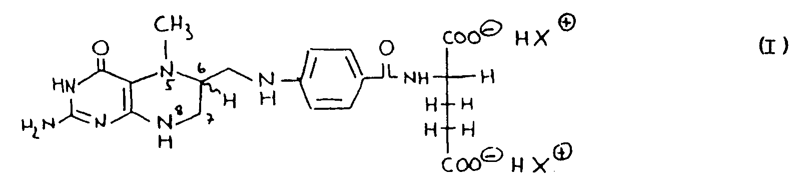 sous la forme du mélange des diastéréoisomères (6RS) ou sous la forme des diastéréoisomères (6R) ou (6S) séparés, dans lesquels
X représente une base contenant de l'azote, choisie de préférence parmi les amines primaires, secondaires et tertiaires ainsi que des acides aminocarboxyliques basiques choisis, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et, en particulier, de la (L)-lysine et de l'arginine et, en particulier, de la (L)-arginine.

2. Procédé de préparation de sels d'ammonium de l'acide (6RS)-N⁵-méthyl-5,6,7,8-tétrahydrofolique de formule I, tels que définis par la revendication 1,
caractérisé en ce que l'on met dans un solvant et, de préférence, dans l'eau, l'acide (6RS)-N⁵-méthyl-5,6,7,8-tétrahydrofolique sous la forme d'un sel de métal alcalin ou de métal alcalino-terreux et, en particulier, de Ca²⁺ ou Mg²⁺;
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine;
que l'on transforme ou que l'on précipite le cation de métal alcalin ou de métal alcalino-terreux contenu dans le sel initial précité, par addition d'un acide approprié et, par exemple, d'acide sulfurique ou d'acide oxalique, sous la forme d'un sel difficilement soluble;
que l'on sépare ensuite le sel difficilement soluble ainsi préparé, par exemple par filtration, et
qu'on élimine finalement le solvant, par exemple par évaporation sous pression réduite ou par lyophilisation.

3. Procédé de préparation de sels d'ammonium de l'acide (6RS)-N⁵-méthyl-5,6,7,8-tétrahydrofolique de formule I, tels que définis par la revendication 1,
caractérisé en ce que l'on met dans au moins un solvant et, de préférence, dans l'eau, l'acide (6RS)-N⁵-méthyl-5,6,7,8-tétrahydrofolique;
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine; et
qu'on élimine finalement le ou les solvants, par exemple par évaporation sous pression réduite ou lyophilisation.

4. Procédé de séparation du mélange des diastéréoisomères (6RS) d'un sel d'ammonium de l'acide N⁵-méthyl-5,6,7,8-tétrahydrofolique de formule I, tel que défini par la revendication 1,
pour obtenir les diastéréoisomères (6R) et (6S) séparés,
caractérisé en ce que l'on mélange le mélange précité des diastéréoisomères (6RS) préparé, par exemple, selon le procédé de la revendication 2 ou de la revendication 3 et, en particulier, sous forme solide, à un mélange de solvants A contenant de l'eau et au moins un solvant miscible avec l'eau et, de préférence, un solvant polaire et, en particulier, des alcools mono- ou polyvalents ayant de 1 à 5 atomes de carbone tels que, par exemple, l'éthanol,
en ce que l'on transforme le mélange ainsi préparé en une solution transparente, par exemple par chauffage à une température comprise entre 40 et 60°C,
que l'on fait cristalliser ensuite un diastéréoisomère dans cette solution transparente et qu'on le sépare,
que l'on dilue ensuite la liqueur mère avec le ou les solvants précités miscibles avec l'eau et que l'on fait cristalliser l'autre diastéréoisomère dans cette solution diluée et qu'on le sépare.

5. Procédé selon la revendication 4, caractérisé en ce que le mélange de solvants A contient de 50 à 95% en volume et, de préférence, de 75 à 95% en volume et, en particulier, 85% en volume d'éthanol, le reste étant de l'eau.

6. Procédé selon l'une des revendications 4 à 5, caractérisé en ce que l'on utilise, pour une partie en poids de mélange de diastéréoisomères (6RS), de 3 à 10 parties en volume et, en particulier, environ 5 parties en volume de mélange de solvants A.

7. Procédé selon l'une des revendications 4 à 6, caractérisé en ce que l'on ensemence la solution transparente avec des germes et que l'on traite éventuellement la solution ensemencée par les ultrasons.

8. Procédé de préparation de sels de métaux alcalins ou de métaux alcalino-terreux de l'acide (6R) ou (6S)-N⁵-méthyl-5,6,7,8-tétrahydrofolique selon les formules IIa ou IIb 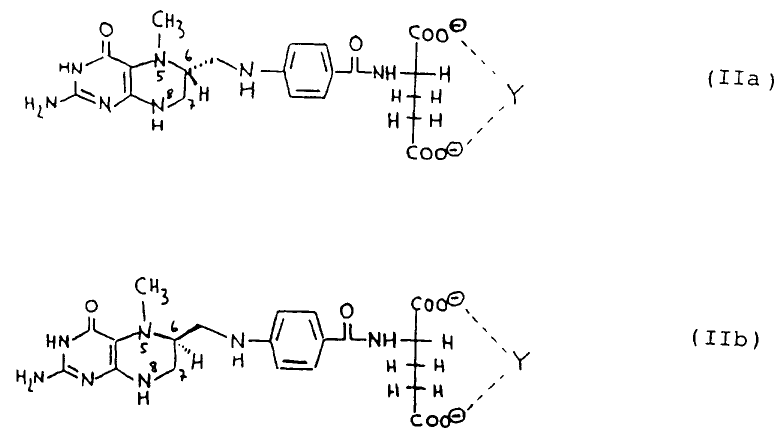 dans lesquelles
Y représente deux cations de métaux alcalins ou un cation de métal alcalino-terreux et, en particulier, de Ca²⁺ ou Mg²⁺,
caractérisé en ce que l'on dissout, dans une partie en volume d'eau, un sel d'ammonium correspondant, formé du diastéréoisomère pur de l'acide N⁵-méthyl-5,6,7,8-tétrahydrofolique de formule Ia ou Ib, 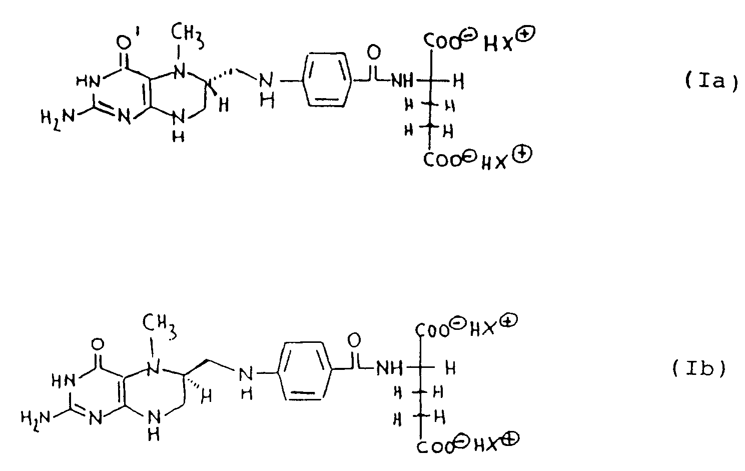 dans lesquelles X est tel que défini par la revendication 1,
préparé, par exemple, selon le procédé de l'une des revendications 4 à 7,
que l'on ajoute ensuite une solution aqueuse d'un sel de métal alcalin ou d'un sel de métal alcalinoterreux et, de préférence, les halogénures correspondants et, en particulier, les chlorures correspondants comme, par exemple, CaCl₂ ou MgCl₂,
que l'on agite ensuite le mélange B ainsi préparé, de préférence a une température comprise dans la gamme de 0°C à 10°C, par exemple pendant environ 1 heure,
que l'on ajoute ensuite de 1,5 à 2,5 parties en volume d'au moins un solvant organique, polaire, miscible avec l'eau et, de préférence, de l'éthanol ou de l'acétone, avec agitation de préférence pendant 30 à 90 minutes, et
que l'on sépare ensuite le sel de métal alcalin ou de métal alcalino-terreux de formule IIa ou IIb ainsi précipité.

9. Procédé selon la revendication 8, caractérisé en ce que l'on règle la valeur du pH du mélange B à une valeur comprise dans la plage de 6,0 à 7,0.

10. Médicament pour le traitement et/ou la lutte contre les tumeurs humaines et/ou animales et/ou pour influencer, de manière synergique, un composé agissant contre le cancer, et/ou pour diminuer la toxicité d'un composé agissant contre le cancer, et/ou pour protéger des cellules humaines et/ou animales, caractérisé en ce qu'il contient au moins comme composé actif, au moins un sel d'ammonium de l'acide N⁵-méthyl-5,6,7,8-tétrahydrofolique de formule I' 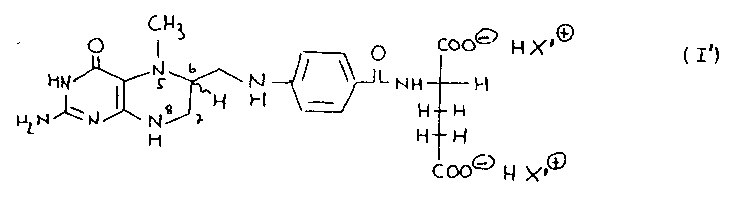 sous la forme du mélange des diastéréoisomères (6RS) ou sous la forme des diastéréoisomères (6R) ou (6S), séparés, dans lequel
X' représente une base contenant de l'azote et pharmacologiquement acceptable, choisie, de préférence, parmi les amines primaires, secondaires et tertiaires, ainsi que parmi les acides aminocarboxyliques basiques et est choisie, en particulier, dans le groupe constitué de l'éthanolamine, de la triéthanolamine, du 2-diméthylaminoéthanol, de la tert-butylamine, de la lysine et en particulier de la (L)-lysine, et de l'arginine et en particulier de la (L)-arginine,
et/ou au moins un sel de métal alcalin et/ou de métal alcalino-terreux de l'acide (6R) ou (6S)-N⁵-méthyl-5,6,7,8-tétrahydrofolique selon les formules IIa ou IIb telles que définies par la revendication 8,
de préférence en quantité comprise entre 1 mg et 500 mg et, plus particulièrement, de 5 mg à 150 mg par unité de dosage et que l'on prépare le médicament de préférence sous la forme d'une préparation pharmaceutique parentérale et/ou orale.

11. Utilisation des sels d'ammonium de l'acide N⁵-méthyl-5,6,7,8-tétrahydrofolique de formule I', telle que définie par la revendication 10,
et/ou des sels de métal alcalin et/ou de métal alcalino-terreux de l'acide (6R) ou (6S)-N⁵-méthyl-5,6,7,8-tétrahydrofolique selon les formules IIa ou IIb telles que définies par la revendication 8,
séparément ou en combinaison quelconque pour la préparation d'un médicament pour le traitement et/ou la lutte contre les tumeurs humaines et/ou animales et/ou pour influencer, de manière synergique, un composé agissant contre le cancer, et/ou pour diminuer la toxicité d'un composé agissant contre le cancer, et/ou pour protéger des cellules humaines et/ou animales, dans lequel ces sels sont présents de préférence dans le médicament, en quantité comprise entre 1 mg et 500 mg, et en particulier entre 5 mg et 150 mg par unité de dosage, et que le médicament se présente, de préférence, sous la forme d'une préparation pharmaceutique parentérale et/ou orale.

## Revendications (Revendications pour l'(les) Etat(s) contractant(s) suivant(s): ES)

1. Procédé de préparation des sels d'ammonium de l'acide (6RS)-N⁵-méthyl-5,6,7,8-tétrahydrofolique de formule I 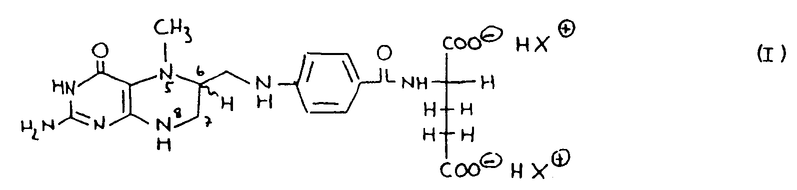 dans laquelle
X représente une base contenant de l'azote, choisie de préférence parmi les amines primaires, secondaires et tertiaires ainsi que des acides aminocarboxyliques basiques choisis, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et, en particulier, de la (L)-lysine et de l'arginine et, en particulier, de la (L)-arginine,
caractérisé en ce que l'on met dans un solvant et, de préférence, dans l'eau, l'acide (6RS)-N⁵-méthyl-5,6,7,8-tétrahydrofolique sous la forme d'un sel de métal alcalin ou de métal alcalino-terreux et, en particulier, de Ca²⁺ ou Mg²⁺;
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine;
que l'on transforme ou que l'on précipite le cation de métal alcalin ou de métal alcalino-terreux contenu dans le sel initial précité, par addition d'un acide approprié et, par exemple, d'acide sulfurique ou d'acide oxalique, sous la forme d'un sel difficilement soluble;
que l'on sépare ensuite le sel difficilement soluble ainsi préparé, par exemple par filtration, et
qu'on élimine finalement le solvant, par exemple par évaporation sous pression réduite ou par lyophilisation.

2. Procédé de préparation des sels d'ammonium de l'acide (6RS)-N⁵-méthyl-5,6,7,8-tétrahydrofolique de formule I, qui ont été définis par la revendication 1,
caractérisé en ce que l'on met, dans au moins un solvant et de préférence dans l'eau, l'acide (6RS)-N⁵-méthyl-5,6,7,8-tétrahydrofolique;
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine; et
qu'on élimine finalement le ou les solvants, par exemple par évaporation sous pression réduite ou lyophilisation.

3. Procédé de séparation du mélange des diastéréoisomères (6RS) d'un sel d'ammonium de l'acide N⁵-méthyl-5,6,7,8-tétrahydrofolique de formule I, tel que défini par la revendication 1,
pour obtenir les diastéréoisomères (6R) et (6S) séparés,
caractérisé en ce que l'on mélange le mélange précité des diastéréoisomères (6RS) préparé, par exemple, selon le procédé de la revendication 1 ou de la revendication 2 et, en particulier, sous forme solide, à un mélange de solvants A contenant de l'eau et au moins un solvant miscible avec l'eau et, de préférence, un solvant polaire et, en particulier, des alcools mono- ou polyvalents ayant de 1 à 5 atomes de carbone tels que, par exemple, l'éthanol,
en ce que l'on transforme le mélange ainsi préparé en une solution transparente, par exemple par chauffage à une température comprise entre 40 et 60°C,
que l'on fait cristalliser ensuite un diastéréoisomère dans cette solution transparente et qu'on le sépare,
que l'on dilue ensuite la liqueur mère avec le ou les solvants précités miscibles avec l'eau et que l'on fait cristalliser l'autre diastéréoisomère dans cette solution diluée et qu'on le sépare.

4. Procédé selon la revendication 3, caractérisé en ce que le mélange de solvants A contient de 50 à 95% en volume et, de préférence, de 75 à 95% en volume et, en particulier, 85% en volume d'éthanol, le reste étant de l'eau.

5. Procédé selon l'une des revendications 3 ou 4, caractérisé en ce que l'on utilise, pour une partie en poids de mélange de diastéréoisomères (6RS), de 3 à 10 parties en volume et, en particulier, environ 5 parties en volume de mélange de solvants A.

6. Procédé selon l'une des revendications 3 à 5, caractérisé en ce que l'on ensemence la solution transparente avec des germes et que l'on traite éventuellement la solution ensemencée par les ultrasons.

7. Procédé de préparation de sels de métaux alcalins ou de métaux alcalino-terreux de l'acide (6R) ou (6S)-N⁵-méthyl-5,6,7,8-tétrahydrofolique selon les formules IIa ou IIb 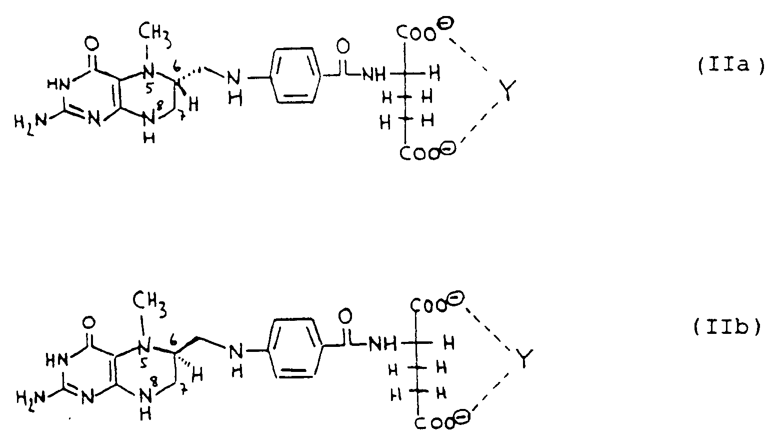 dans lesquelles
Y représente deux cations de métal alcalin ou un cation de métal alcalino-terreux et, en particulier, Ca²⁺ ou Mg²⁺,
caractérisé en ce que l'on dissout, dans une partie en volume d'eau, un sel d'ammonium correspondant, formé de diastéréoisomère pur, de l'acide N⁵-méthyl-5,6,7,8-tétrahydrofolique de formule Ia ou Ib, 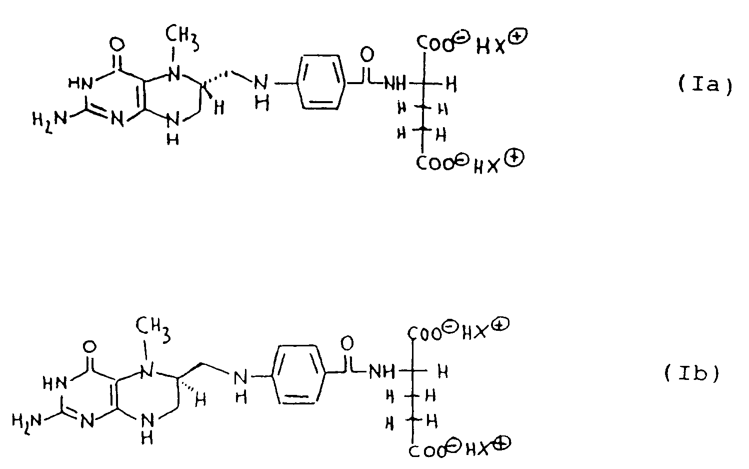 dans lesquelles X est tel que défini par la revendication 1,
préparé par exemple selon le procédé de l'une des revendications 3 à 6,
que l'on ajoute ensuite une solution aqueuse d'un sel de métal alcalin ou d'un sel de métal alcalinoterreux et, de préférence, les halogénures correspondants et, en particulier, les chlorures correspondants comme, par exemple, CaCl₂ ou MgCl₂,
que l'on agite ensuite le mélange B ainsi préparé, de préférence à une température comprise dans la gamme de 0°C à 10°C, par exemple pendant environ 1 heure,
que l'on ajoute ensuite de 1,5 à 2,5 parties en volume d'au moins un solvant organique, polaire, miscible avec l'eau et, de préférence, de l'éthanol ou de l'acétone, avec agitation de préférence pendant 30 à 90 minutes, et
que l'on sépare ensuite le sel de métal alcalin ou de métal alcalino-terreux de formule IIa ou IIb ainsi précipité.

8. Procédé selon la revendication 7, caractérisé en ce que l'on règle la valeur du pH du mélange B à une valeur comprise dans la plage de 6,0 à 7,0.

9. Procédé de préparation de médicaments pour le traitement et/ou la lutte contre les tumeurs humaines et/ou animales et/ou pour influencer, de manière synergique, un composé agissant contre le cancer, et/ou pour diminuer la toxicité d'un composé agissant contre le cancer, et/ou pour protéger des cellules humaines et/ou animales, caractérisé en ce que l'on ajoute au médicament, outre les adjuvants et supports usuels, comme au moins un composé actif, au moins un sel selon les revendications précédentes, de préférence en quantité de 1 mg à 500 mg et, en particulier, de 5 mg à 150 mg par unité de dosage, dans lequel le médicament se présente sous la forme d'une préparation pharmaceutique parentérale et/ou orale et est administré comme tel.

## Revendications (Revendications pour l'(les) Etat(s) contractant(s) suivant(s): GR)

1. Sels d'ammonium de l'acide N⁵-méthyl-5,6,7,8-tétrahydrofolique de formule I 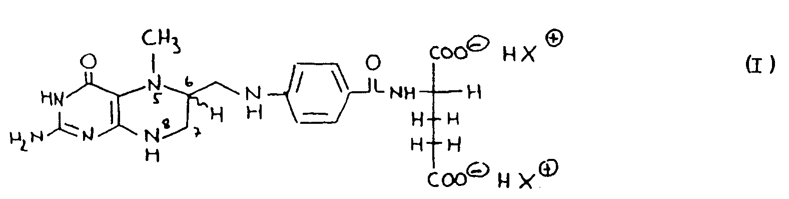 sous la forme du mélange des diastéréoisomères (6RS) ou sous la forme des diastéréoisomères (6R) ou (6S) séparés, dans lesquels
X représente une base contenant de l'azote, choisie de préférence parmi les amines primaires, secondaires et tertiaires ainsi que des acides aminocarboxyliques basiques choisis, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et, en particulier, de la (L)-lysine et de l'arginine et, en particulier, de la (L)-arginine, qui ont été préparés selon la revendication 2 ou 3.

2. Procédé de préparation de sels d'ammonium de l'acide (6RS)-N⁵-méthyl-5,6,7,8-tétrahydrofolique de formule I, tels que définis par la revendication 1,
caractérisé en ce que l'on met dans un solvant et, de préférence, dans l'eau, l'acide (6RS)-N⁵-méthyl-5,6,7,8-tétrahydrofolique sous la forme d'un sel de métal alcalin ou de métal alcalino-terreux et, en particulier, de Ca²⁺ ou Mg²⁺;
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine;
que l'on transforme ou que l'on précipite le cation de métal alcalin ou de métal alcalino-terreux contenu dans le sel initial précité, par addition d'un acide approprié et, par exemple, d'acide sulfurique ou d'acide oxalique, sous la forme d'un sel difficilement soluble;
que l'on sépare ensuite le sel difficilement soluble ainsi préparé, par exemple par filtration, et
qu'on élimine finalement le solvant, par exemple par évaporation sous pression réduite ou par lyophilisation.

3. Procédé de préparation de sels d'ammonium de l'acide (6RS)-N⁵-méthyl-5,6,7,8-tétrahydrofolique de formule I, tels que définis par la revendication 1,
caractérisé en ce que l'on met dans au moins un solvant et, de préférence, dans l'eau, l'acide (6RS)-N⁵-méthyl-5,6,7,8-tétrahydrofolique,
que l'on y ajoute ensuite une base contenant de l'azote, de préférence en quantité comprise entre 2 et 2,5 équivalents molaires, ladite base étant choisie, de préférence, parmi les amines primaires, secondaires et tertiaires, ainsi que parmi les acides aminocarboxyliques basiques et est choisie, en particulier, dans le groupe constitué de la cyclohexylamine, de la diisopropylamine, de la benzylamine, de l'ammoniaque, de l'éthanolamine, de la triéthanolamine, du 2-diméthylaminoéthanol, de la tert-butylamine, de la lysine et en particulier de la (L)-lysine, et de l'arginine et en particulier de la (L)-arginine;et
qu'on élimine finalement le ou les solvants par exemple par évaporation sous pression réduite ou par lyophilisation.

4. Procédé de séparation du mélange des diastéréoisomères (6RS) d'un sel d'ammonium de l'acide N⁵-méthyl-5,6,7,8-tétrahydrofolique de formule I, tel que défini par la revendication 1,
pour obtenir les diastéréoisomères (6R) et (6S) séparés,
caractérisé en ce que l'on mélange le mélange précité des diastéréoisomères (6RS) préparé, par exemple, selon le procédé de la revendication 2 ou de la revendication 3 et, en particulier, sous forme solide, à un mélange de solvants A contenant de l'eau et au moins un solvant miscible avec l'eau et, de préférence, un solvant polaire et, en particulier, des alcools mono- ou polyvalents ayant de 1 à 5 atomes de carbone tels que, par exemple, l'éthanol,
en ce que l'on transforme le mélange ainsi préparé en une solution transparente, par exemple par chauffage à une température comprise entre 40 et 60°C,
que l'on fait cristalliser ensuite un diastéréoisomère dans cette solution transparente et qu'on le sépare,
que l'on dilue ensuite la liqueur mère avec le ou les solvants précités miscibles avec l'eau et que l'on fait cristalliser l'autre diastéréoisomère dans cette solution diluée et qu'on le sépare.

5. Procédé selon la revendication 4, caractérisé en ce que le mélange de solvants A contient de 50 à 95% en volume et, de préférence, de 75 à 95% en volume et, en particulier, 85% en volume d'éthanol, le reste étant de l'eau.

6. Procédé selon l'une des revendications 4 à 5, caractérisé en ce que l'on utilise, pour une partie en poids de mélange de diastéréoisomères (6RS), de 3 à 10 parties en volume et, en particulier, environ 5 parties en volume de mélange de solvants A.

7. Procédé selon l'une des revendications 4 à 6, caractérisé en ce que l'on ensemence la solution transparente avec des germes et que l'on traite éventuellement la solution ensemencée par les ultrasons.

8. Procédé de préparation de sels de métaux alcalins ou de métaux alcalino-terreux de l'acide (6R) ou (6S)-N⁵-méthyl-5,6,7,8-tétrahydrofolique de formule IIa ou IIb 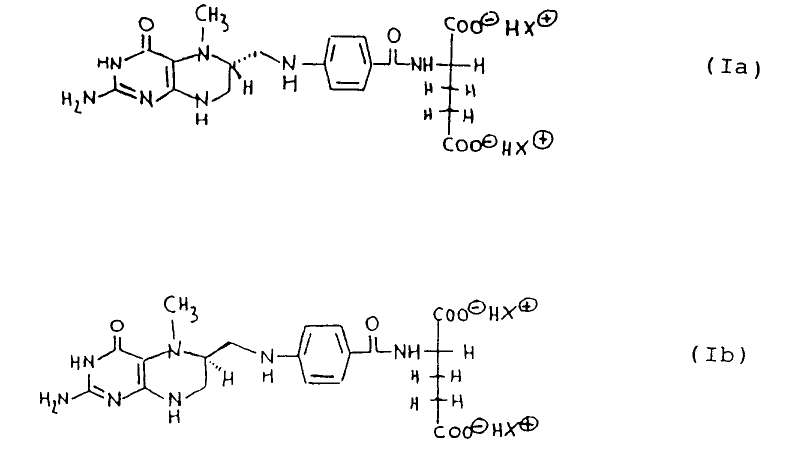 dans lesquelles
Y représente deux cations de métaux alcalins ou un cation de métal alcalino-terreux et, en particulier, de Ca²⁺ ou Mg²⁺,
caractérisé en ce que l'on dissout, dans une partie en volume d'eau, un sel d'ammonium correspondant, formé de diastéréoisomère pur de l'acide N⁵-méthyl-5,6,7,8-tétrahydrofolique de formule Ia ou Ib, 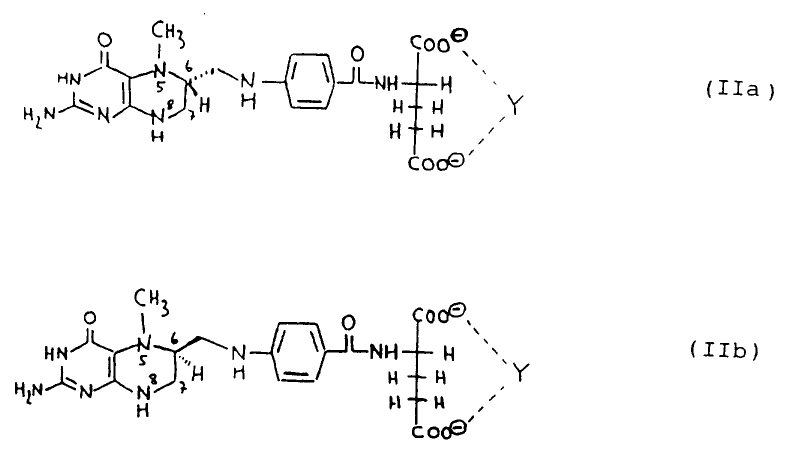 dans lesquelles X est tel que défini par la revendication 1,
préparé par exemple selon le procédé de l'une des revendications 4 à 7,
que l'on ajoute ensuite une solution aqueuse d'un sel de métal alcalin ou d'un sel de métal alcalino-terreux et, de préférence, les halogénures correspondants et, en particulier, les chlorures correspondants comme, par exemple, CaCl₂ ou MgCl₂,
que l'on agite ensuite le mélange B ainsi préparé, de préférence à une température comprise dans la gamme de 0°C à 10°C, par exemple pendant environ 1 heure,
que l'on ajoute ensuite de 1,5 à 2,5 parties en volume d'au moins un solvant organique, polaire, miscible avec l'eau et, de préférence, de l'éthanol ou de l'acétone, avec agitation de préférence pendant 30 à 90 minutes, et
que l'on sépare ensuite le sel de métal alcalin ou de métal alcalino-terreux de formule IIa ou IIb ainsi précipité.

9. Procédé selon la revendication 8, caractérisé en ce que l'on règle la valeur du pH du mélange B à une valeur comprise dans la plage de 6,0 à 7,0.

10. Utilisation des sels d'ammonium de l'acide N⁵-méthyl-5,6,7,8-tétrahydrofolique de formule I' 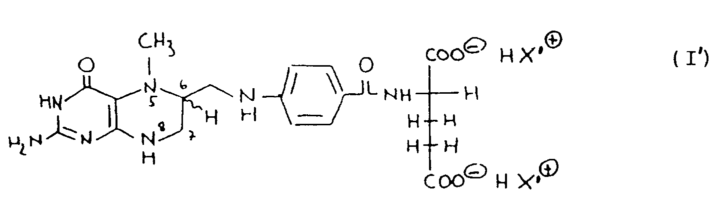 sous la forme du mélange des diastéréoisomères-(6RS) ou sous la forme des diastéréoisomères-(6R) ou (6S) séparés, dans lequel
X' représente une base contenant de l'azote et pharmacologiquement acceptable, choisie, de préférence, parmi les amines primaires, secondaires et tertiaires, ainsi que parmi les acides aminocarboxyliques basiques et est choisie, en particulier, dans le groupe constitué de l'éthanolamine, de la triéthanolamine, du 2-diméthylaminoéthanol, de la tert-butylamine, de la lysine et en particulier de la (L)-lysine, et de l'arginine et en particulier de la (L)-arginine, qui ont été préparés selon la revendication 2 ou 3,
et/ou des sels de métal alcalin et/ou de métal alcalino-terreux de l'acide (6R) ou (6S)-N⁵-méthyl-5,6,7,8-tétrahydrofolique selon les formules IIa ou IIb telles que définies par la revendication 8,
séparément ou en combinaison quelconque pour la préparation d'un médicament pour le traitement et/ou la lutte contre les tumeurs humaines et/ou animales et/ou pour influencer, de manière synergique, un composé agissant contre le cancer, et/ou pour diminuer la toxicité d'un composé agissant contre le cancer, et/ou pour protéger des cellules humaines et/ou animales, dans lequel ces sels sont présents, de préférence en quantité comprise entre 1 mg et 500 mg, et plus particulièrement entre 5 mg et 150 mg par unité de dosage et que le médicament se présente de préférence sous la forme d'une préparation pharmaceutique parentérale et/ou orale.
